# EUROPEAN PATENT APPLICATION

(11) **EP 1 698 611 A1**
(43) Date of publication of application: **06.09.2006**
(21) Application number: 04807933.9
(22) Date of filing: 27.12.2004
(51) Int. Cl.: C07C 201/12, C07C 205/56, C07C 229/40, C07D 209/42, C07D 403/12

(54) **PROCESS FOR PRODUCING PHENYLACETIC ACID DERIVATIVE**

(30) Priority: 26.12.2003 JP 2003431680; 29.09.2004 JP 2004283082; 27.10.2004 JP 2004312335
(71) Applicant: DAIICHI PHARMACEUTICAL CO., LTD., Chuo-ku, Tokyo 103-8234 (JP)
(72) Inventor: NAKAYAMA, Atsushi c/o Daiichi Pharmaceut.Co. Ltd., Edogawa-ku, Tokyo 1348630 (JP); NOGUCHI, Shigeru c/o Daiichi Pharmaceut. Co. Ltd., Edogawa-ku, Tokyo 1348630 (JP); FURUYA, Yukito c/o Daiichi Pharmaceut. Co. Ltd., Edogawa-ku, Tokyo 1348630 (JP); OKANO, Katsuhiko c/o Daiichi Pharmaceut. Co. Ltd., Edogawa-ku, Tokyo 1348630 (JP)
(74) Representative: Hartz, Nikolai
(86) International application number: PCT/JP2004/019578
(87) International publication number: WO 2005/063678

(57) **Abstract**

The present invention provides an advantageous process for producing an intermediate which is useful for producing a compound which exhibits excellent VLA-4 inhibitory effect and safety.

Intermediates (III) and (XIII) are produced through conversion through the following reaction schemes.

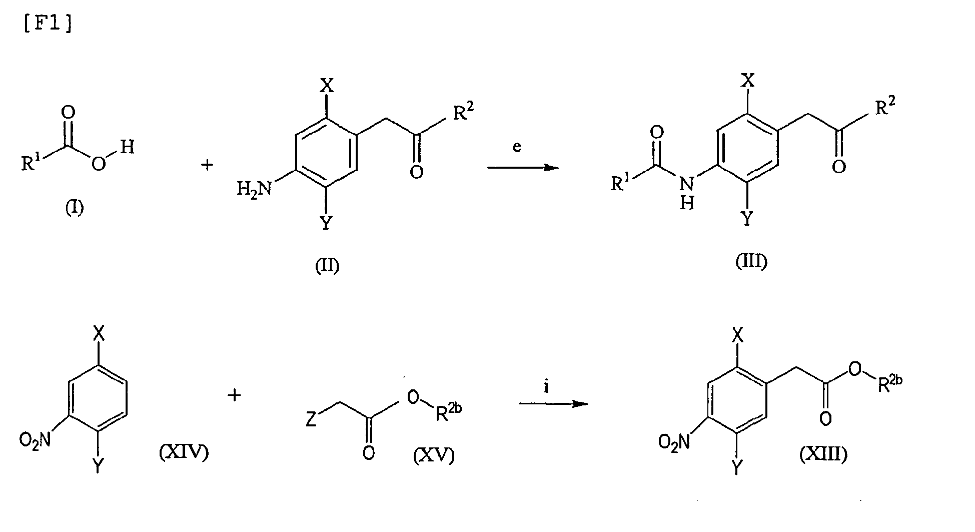

## Description

### Technical Field

The present invention relates to a process for producing a compound which is useful as an intermediate for production of a compound which exhibits excellent inhibitory effect against Very-Late-Antigen-4 (VLA-4) and safety.

### Background Art

VLA-4 is a molecule involved in cell adhesion and is expressed on monocytes, lymphocytes, eosinophils, and basophils. It is known that VLA-4 plays a role as a receptor for Vascular Cell Adhesion Molecule-1 (VCAM-1) or a similar molecule.

Recently, it has been reported that selective inhibition of cell adhesion mediated via VLA-4 and VCAM-1 may be a resolution method for the treatment of autoimmune diseases and allergic inflammatory diseases.

A compound represented by formula (I) described in Patent Document 1; e.g., a compound represented by the following formula (1), is a promising drug compound which exhibits anti-inflammatory effect based on its excellent VLA-4 inhibitory effect and highly safety (see Patent Document 1).

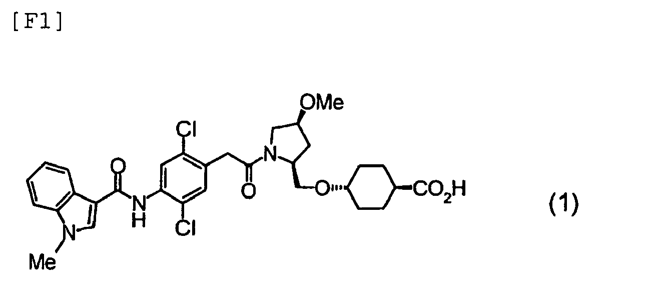

A compound represented by the following formula (2):

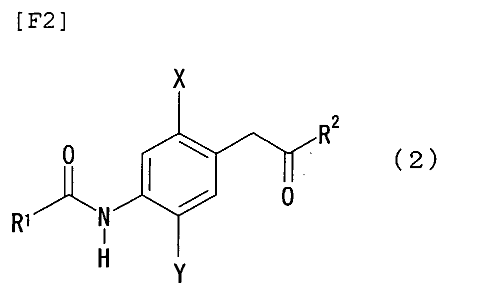

(wherein R¹ represents an aryl group which may be substituted, or a heteroaryl group which may be substituted; R² represents a linear or branched lower alkoxy group which may be substituted, an aralkyloxy group which may be substituted, a phenoxy group, or a moiety represented by formula (a):

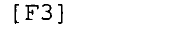

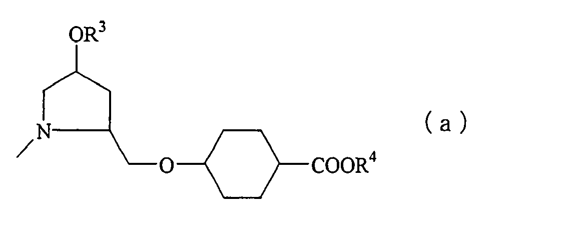

(wherein R³ represents a linear or branched lower alkyl group which may be substituted; and R⁴ represents a linear or branched lower alkyl group which may be substituted, or an aralkyl group which may be substituted), X represents a hydrogen atom or a halogen atom, and Y represents a halogen atom or a lower alkoxy group) and a compound represented by formula (13):

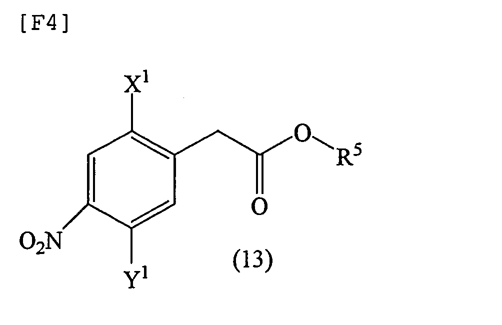

(wherein X¹ represents a hydrogen atom or a halogen atom, Y¹ represents a halogen atom, R⁵ represents a hydrogen atom, a linear or branched alkyl group which may be substituted, an aralkyl group which may be substituted, or a phenyl group) are important intermediates in the production of the compound represented by formula (I) described in Patent Document 1.

Compounds represented by formula (2) in which R² is an alkoxy group or an aralkyloxy group are known to be produced via esterifying a carboxylic moiety of a substituted or non-substituted nitrophenylacetic acid, reducing the nitro group to form an amino group, and condensing the product with a carboxylic acid having R¹ as a partial structure or an ester thereof, to thereby form an amide (see, Patent Documents 1 and 2).
However, according to the production methods as described in Patent Documents 1 and 2, reduction of the nitro group is performed in the presence of metallic iron. Thus, operativity and reactivity are not satisfactory. In addition, since amidation in the above step is performed under conventional basic conditions, a dimer of the carboxylic acid having R¹ as a partial structure is formed by side reaction, resulting in a considerable loss in yield of main reaction product. The thus-formed dimer product must be removed through column chromatography.
Compounds represented by formula (2) in which R² is a moiety represented by formula (a) are produced via condensation of a carboxylic acid having R¹ as a partial structure or an ester thereof with aminophenylacetamide. The amidation, which is also performed under conventional basic conditions, raises the same problem as mentioned above.

The compound represented by formula (13) in which R⁵ is a hydrogen atom and each of X¹ and Y¹ is a chlorine atom is disclosed be produced via, for example, brominating the methyl group of 2,5-dichloro-nitrotoluene, cyanating 2,5-dichloro-4-nitrobenzylbromide so as to form 2,5-dichloro-4-nitrophenylacetonitrile, and hydrolyzing the cyano group, to thereby form 2,5-dichloro-4-nitrophenylacetic acid. However, this method includes a number of steps, which is problematic (see Non-Patent Document 1).

A method for producing 2,5-dichloro-4-nitrophenylmalonic acid ester derivatives, which is an analogous of compounds represented by formula (13), is also disclosed (see Patent Document 3). In the reported method, 1,4-dichloro-2-fluorobenzene is nitrated, and the nitro compound is reacted with malonic acid. However, the method including reaction with malonic acid has problems in that a number of steps must be performed, that expensive 1,4-dichloro-2-fluorobenzene is employed, and that lithium hydride, which is not easily handled on an industrial scale, is employed.

Also disclosed are introduction of -P(O) (OCH₃)₂, -SO₂Ph, -CN, or -CO₂R (R represents a lower alkyl group) serving as a side chain group to nitrobenzene (see Non-Patent Document 2), introduction of ethyl chloroacetate or a similar compound to nitrobenzene (see Non-Patent Document 3). However, according to these reactions, products are predominantly orthosubstituted nitrobenzenes.

A reaction for introducing an appropriate substituent at a para position with respect to the nitro group of nitrobenzene is also disclosed (see Non-Patent Document 4). In the method, a variety of nitroarenes are reacted with a variety of 2-chloropropionate esters, to thereby synthesize nitroarylpropionates. In the reaction, non-substituted or mono-substituted (by, for example, a halogen atom) nitrobenzene is employed. However, the document does not disclose reaction of di-halo-substituted nitrobenzene.
[Patent Document 1] WO2002/053534
[Patent Document 2] PCT/JP2004/006471
[Patent Document 3] JP-B-3350051
[Patent Document 4] JP-A-1982-16840
[Non-Patent Document 1] Ann. Appl. Biol., 69, 65-72 (1971)
[Non-Patent Document 2] J. Amer. Chem. Soc., 107, 19, 5473-5483 (1985)
[Non-Patent Document 3] Synthesis, 12, 1007-1009 (1988)
[Non-Patent Document 4] J. Org. Chem., 49, 578-579 (1984)

### Disclosure of the Invention

### Problems to be Solved by the Invention

The present invention is directed to provide an industrially advantageous process for producing compounds represented by the above formulas (2) and (13), the compounds being important as intermediates in the production of a compound which exhibits excellent VLA-4 inhibitory effect and safety

### Means for Solving the Problems

The present inventors have conducted extensive studies, and have found that, in the step of producing a compound represented by formula (2), the nitro group can be selectively reduced without reducing a halogen on the benzene ring in the presence of a platinum-based catalyst under mild conditions. The inventors have also found that the stability of the reduction product can be enhanced through production in the hydrochloride crystal form.
The inventors have also found that amidation between a compound which can form an acid chloride and an amine proceeds quantitatively under acidic conditions instead of under conventional base-added conditions, whereby compound (2) can be produced at high yield.

The inventors have also found that a method for
producing an intermediate (13) for a compound which inhibits cell adhesion caused by VLA-4/VCAM-1 interaction in a single step at low cost, the method including, in the step of producing the compound represented by formula (13), introducing an ester derivative into readily available substituted nitrobenzene in the presence of a base. Surprisingly, the inventors have found that a substituent can be introduced into a para position of the nitrobenzene ring with respect to the N-bonded group at a selectivity as high as 90%.

The present invention has been accomplished on the basis of these excellent findings.

The present invention provides a process for producing a compound represented by formula (III): 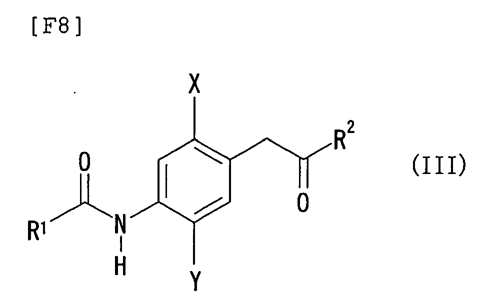 (wherein R¹ represents an aryl group which may be substituted, or a heteroaryl group which may be substituted; R² represents a linear or branched lower alkoxy group which may be substituted, an aralkyloxy group which may be substituted, a phenoxy group, or a group represented by formula (a): 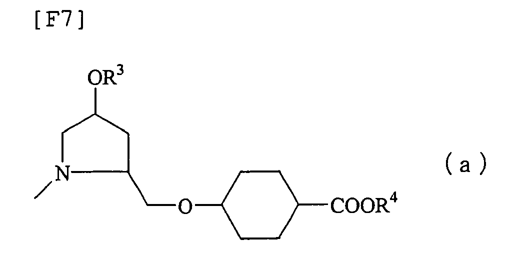 (wherein R³ represents a linear or branched lower alkyl group which may be substitued; and R⁴ represents a linear or branched lower alkyl group which may be substituted, or an aralkyl group which may be substituted); X represents a hydrogen atom or a halogen atom; and Y represents a halogen atom or a lower alkoxy group), characterized by comprising reacting a compound represented by formula (I): 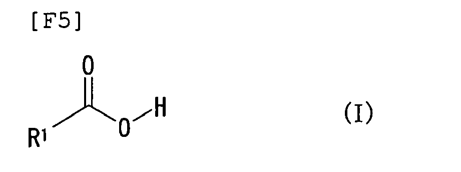 (wherein R¹ has the same meaning as defined above) with a chlorinating agent and a compound represented by formula (II) : 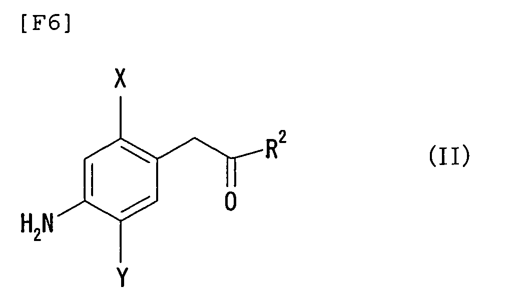 (wherein R² has the same meaning as defined above) or a salt thereof under acidic conditions without addition of a base.

The present invention also provides a hydrochloric acid salt of a compound represented by formula (IV).

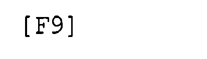

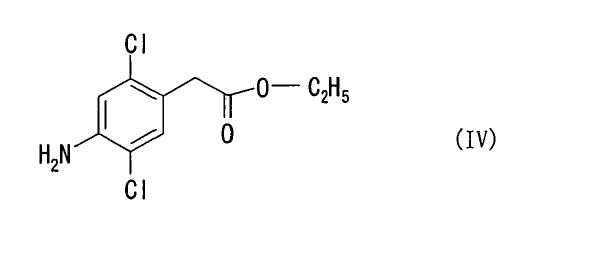

The present invention also provides a process for producing a compound represented by formula (VII): 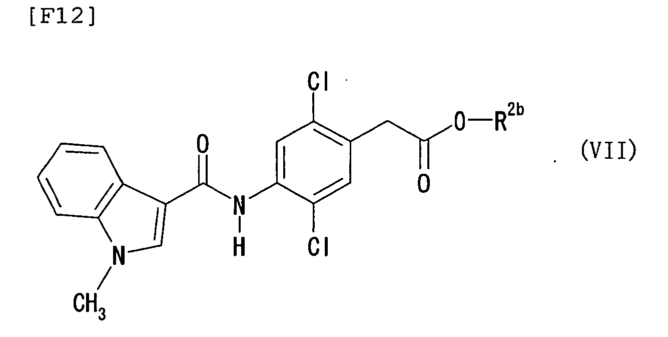 (wherein R^{2b} represents a linear or branched lower alkyl group which may be substituted, an aralkyl group which may be substituted, or a phenyl group), characterized by comprising reacting a compound represented by formula (V): 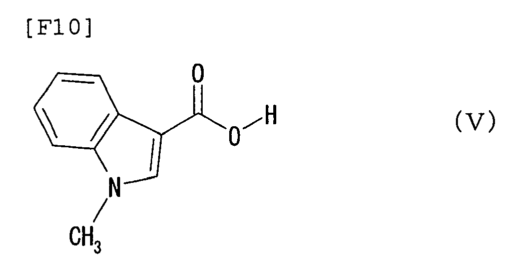 with a chlorinating agent and a compound represented by formula (VI): 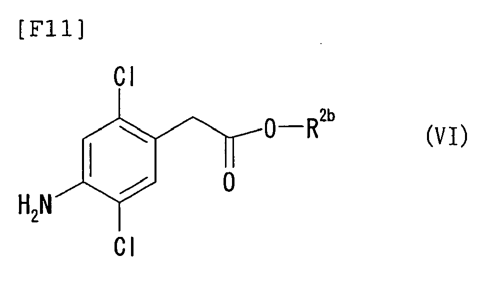 (wherein R^{2b} has the same meaning as defined above) or a salt thereof under acidic conditions without addition of a base.

The present invention also provides a process for producing a compound represented by formula (IX): 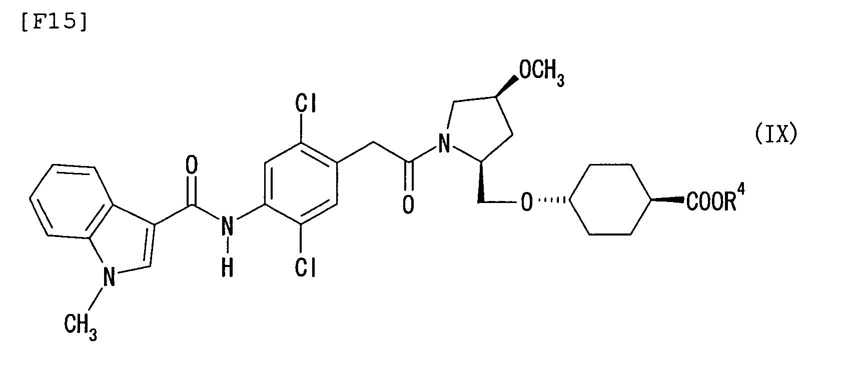 (wherein R⁴ has the same meaning as defined above), characterized by comprising reacting a compound represented by formula (V): 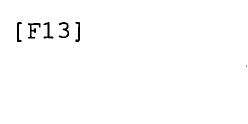 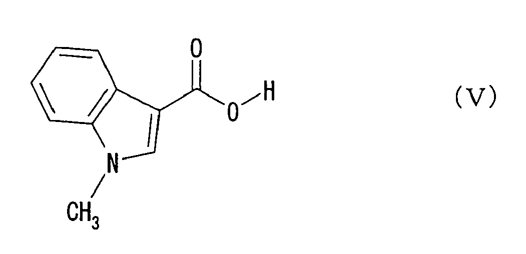 with a chlorinating agent and a compound represented by formula (VIII): 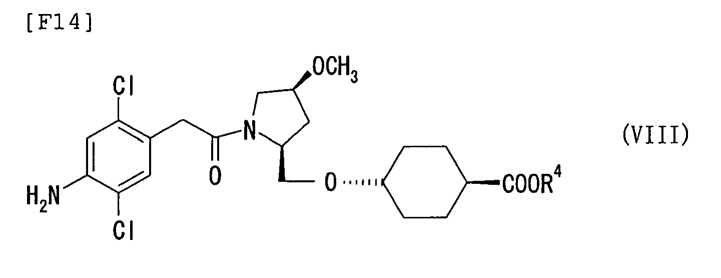 (wherein R⁴ has the same meaning as defined above) or a salt thereof under acidic conditions without addition of a base.

The present invention also provides a process for producing a compound represented by formula (XII): 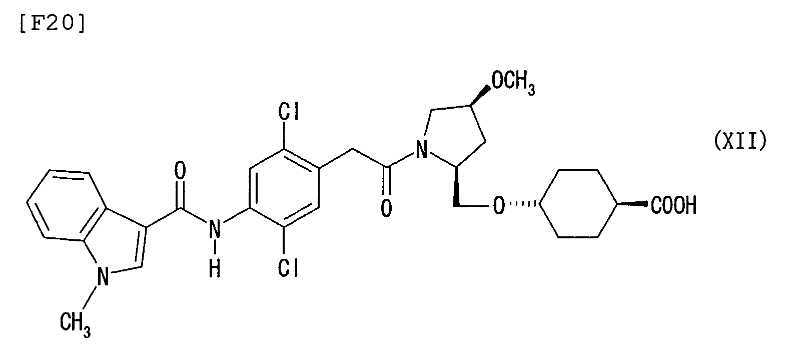 or a salt thereof, or a hydrate of the compound or the salt,
characterized by comprising reacting a compound represented by formula (V): 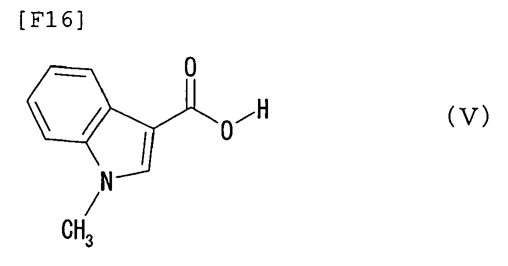 with a chlorinating agent and a compound represented by formula (VI): 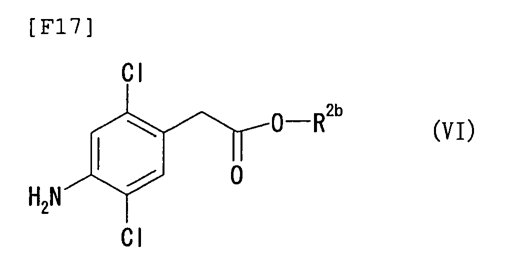 (wherein R^{2b} represents a linear or branched lower alkyl group which may be substituted, or an aralkyl group which may be substituted, or a phenyl group) or a salt thereof under acidic conditions without addition of a base; optionally hydrolyzing the product to thereby yield a compound represented by formula (X): 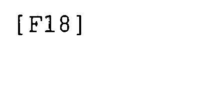 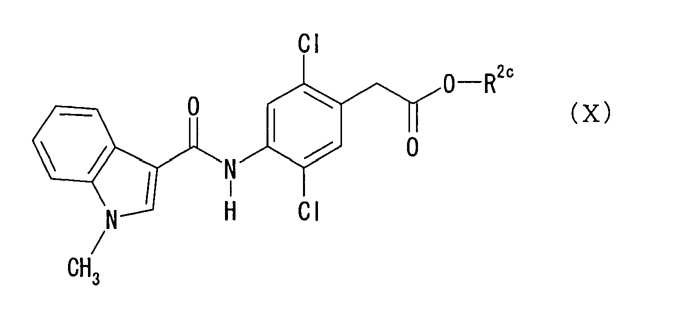 (wherein R^{2c} represents a hydrogen atom, a linear or branched lower alkyl group which may be substituted, an aralkyl group which may be substituted, or a phenyl group); reacting the compound represented by formula (X) with a compound represented by formula (XI): 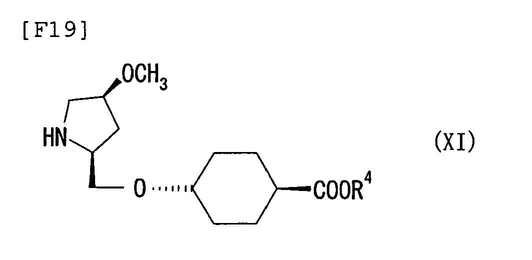 (wherein R⁴ represents a linear or branched lower alkyl group which may be substituted, or an aralkyl group which may be substituted); and hydrolyzing the product.

The present invention also provides a process for producing a compound represented by formula (XII): 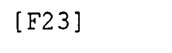 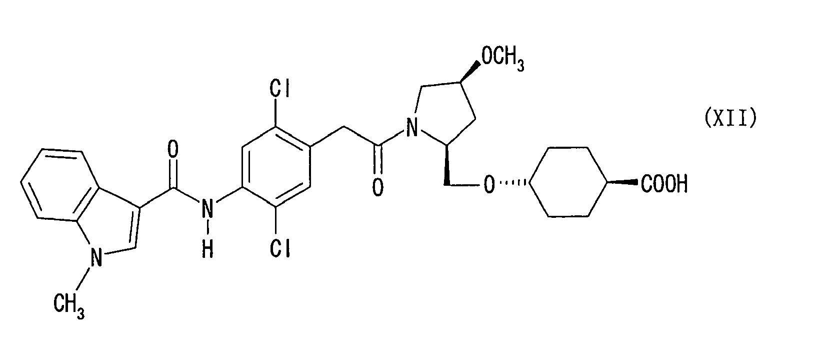 or a salt thereof, or a hydrate of the compound or the salt, characterized by comprising reacting a compound represented by formula (V): 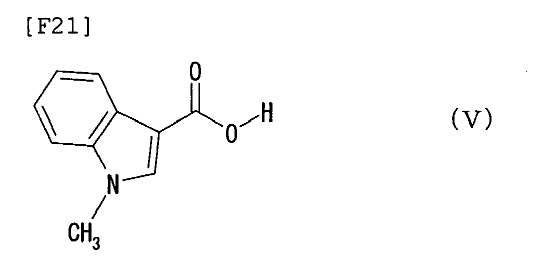 with a chlorinating agent and a compound represented by formula (VIII): 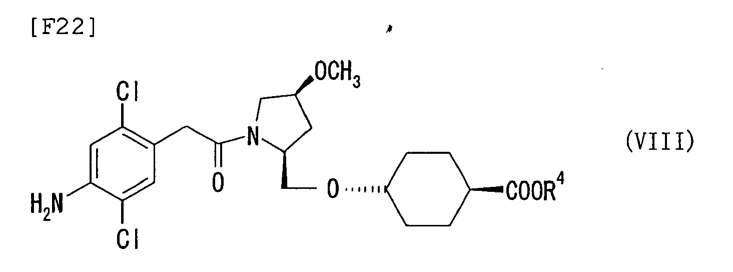 (wherein R⁴ represents a linear or branched lower alkyl group which may be substituted, or an aralkyl group which may be substituted) or a salt thereof under acidic conditions without addition of a base; and hydrolyzing the product.

The present invention also provides a process for producing a compound represented by formula (XIII): 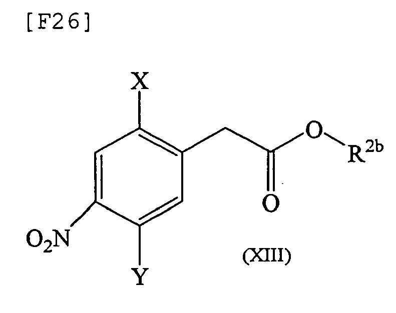 (wherein X, Y, and R^{2b} have the same meanings as defined above), characterized by comprising reacting a compound represented by formula (XIV): 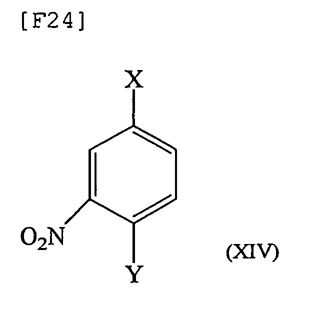 (wherein X and Y have the same meanings as defined above) with a compound represented by formula (XV): 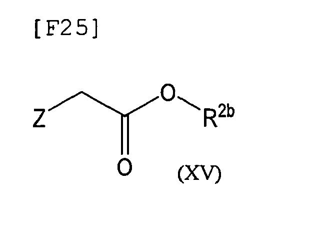 (wherein Z represents a halogen atom, a phenylthio group, an alkoxy group, or an amino group; and R^{2b} has the same meaning as defined above) in a solvent in the presence of a base.
The present invention also provides a process for producing the compound represented by formula (III) according to the above process, wherein the compound represented by formula (II) is produced through reduction of the nitro group of the compound represented by formula (XIII) produced through the above process.

### Effects of the Invention

The processes of the present invention facilitate efficient production of a drug compound represented by formula (I) described in Patent Document 1, the compound exhibiting anti-inflammatory effect based on its excellent VLA-4 inhibitory effect and being highly safety.

### Best Modes for Carrying Out the Invention

The present invention will next be described in detail.
In one preferred embodiment, the present invention employs the following reaction scheme starting with an easily available substituted nitrophenylacetic acid. Alternatively, an easily available substituted nitrobenzene may also be employed as a starting material.

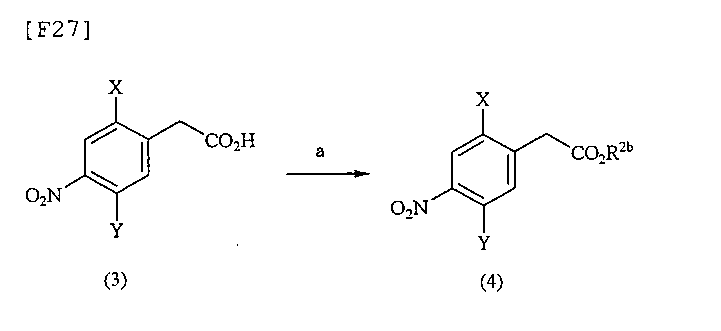

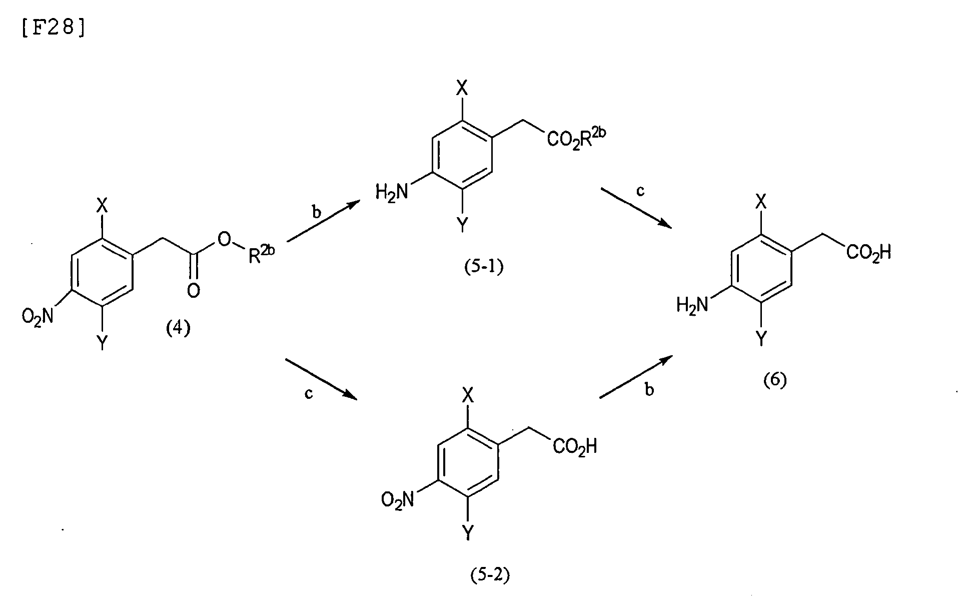

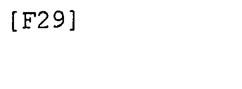

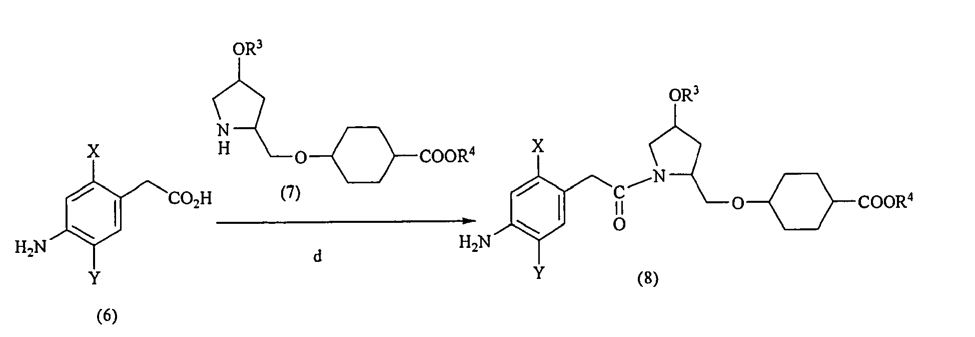

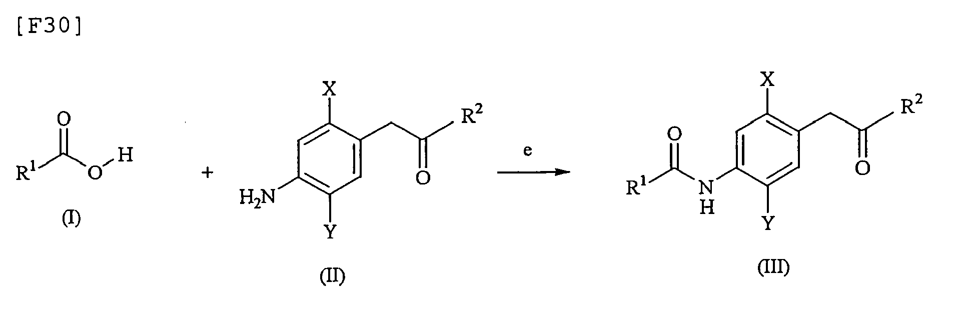

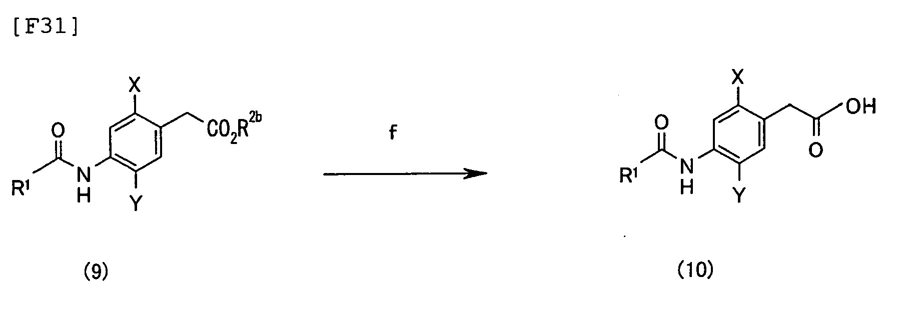

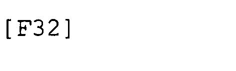

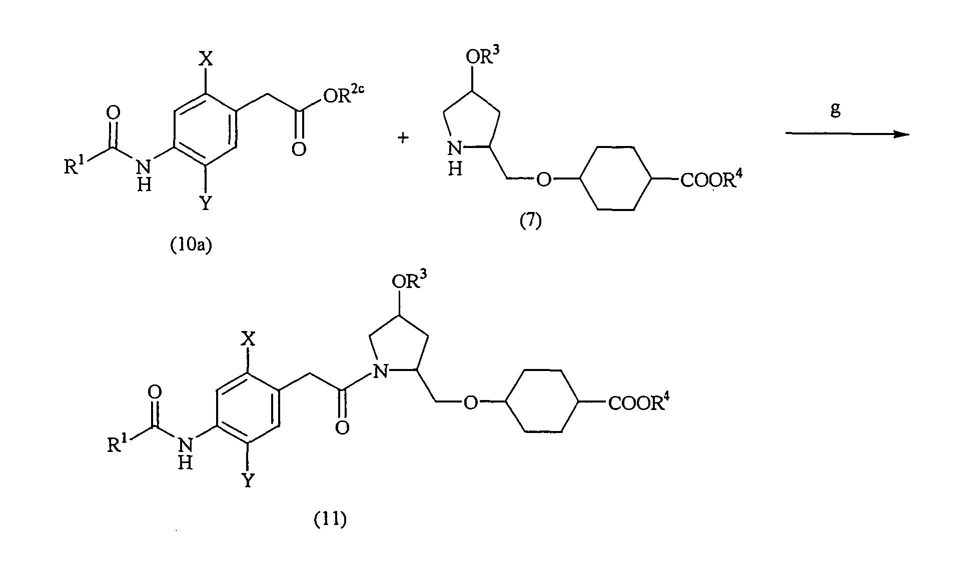

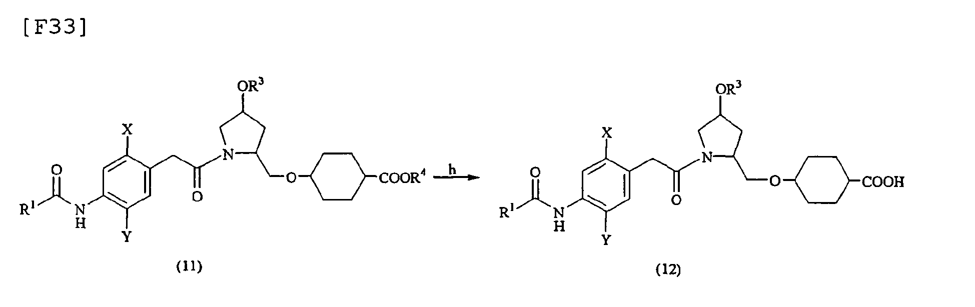

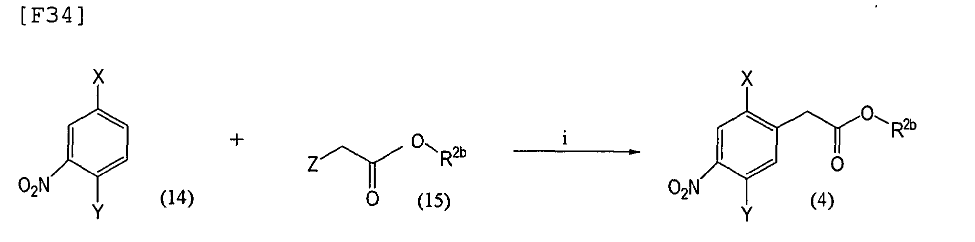

In the above reaction schemes, R^{2b} represents a linear or branched lower alkyl group which may be substituted, an aralkyl group which may be substituted, or a phenyl group; R^{2c} represents a hydrogen atom, a linear or branched lower alkyl group which may be substituted, an aralkyl group which may be substituted, or a phenyl group; and R¹, R², R³, R⁴, X, Y, and Z have the same meanings as defined above.

In the above reaction schemes, the compound represented by formula (II) includes compounds represented by formulas (5-1) and (8), and the compound represented by formula (III) includes compounds represented by formulas (9), (10a), and (11).
Each step will next be described in detail.
[Step a]

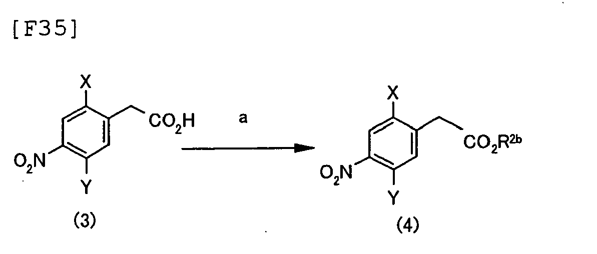

In step a, a carboxylic acid represented by formula (3) is converted to an ester represented by formula (4).

Step a may be performed in accordance with a known method for converting a carboxylic acid to an ester (see "Protective Groups in Organic Synthesis," edited by T. W. Greene and P. G. Wuts, John Wiley&Sons, Inc., New York, 1991).

In formula (4), R^{2b} represents a linear or branched lower alkyl group which may be substituted, an aralkyl group which may be substituted, or a phenyl group. Examples of the lower alkyl group include C1-C6 alkyl groups. The aralkyl group is preferably a phenyl-C₁₋₆ alkyl group. Examples of the substituent which may be bound to the alkyl group or the aralkyl group include halogen atoms and C1-C6 alkoxy groups, and the number of the substituents may be 1 to 3. Preferred examples of R^{2b} include methyl, ethyl, tert-butyl, benzyl, and 4-methoxybenzyl, with methyl and ethyl being more preferred.

In formulas (3) and (4), X represents a hydrogen atom or a halogen atom, and Y represents a halogen atom or a lower alkoxy group. Examples of the lower alkoxy group include C1-C6 alkoxy groups. Preferably, X and Y each independently represent a fluorine atom or a chlorine atom. More preferably, each of X and Y represents a chlorine atom.

### [Steps b and c]

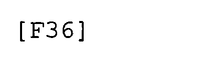

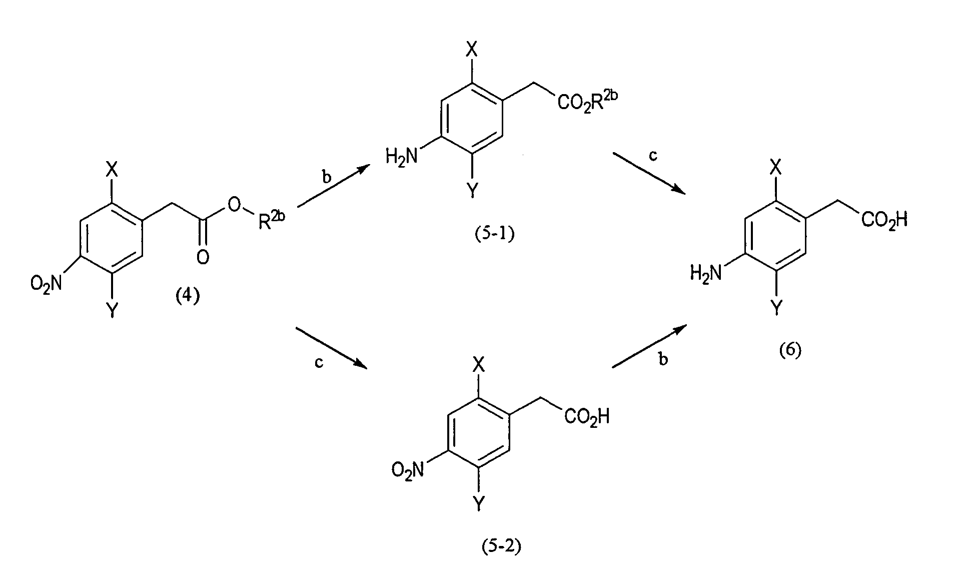

In step b, a nitro group is reduced to an amino group. In step c, an ester is hydrolyzed to a carboxylic acid.
In formulas (4), (5), and (6), R^{2b}, X, and Y have the same meanings as defined above in relation to step a.

In the present invention, in order to synthesize a compound represented by formula (6) from a compound represented by formula (4), any of the following processes may be employed. Specifically, the nitro group of the compound represented by formula (4) is reduced to an amino group to thereby produce a compound represented by formula (5-1), and the ester moiety of the compound represented by formula (5-1) is hydrolyzed to thereby produce a compound represented by formula (6). Alternatively, the ester moiety of the compound represented by formula (4) is hydrolyzed to thereby produce a compound represented by formula (5-2), and the nitro group of the compound represented by formula (5-2) is reduced to an amino group to thereby produce a compound represented by formula (6).

A reduction step similar to step b is described in WO2002/053534, in which an ethyl ester compound (R^{2b} represents an ethyl group) is reduced through addition of sodium acetate and iron powder to the compound in a solvent (ethanol:water = 1:4) and subsequent stirring for about 1 hour at 100°C.

The present inventors have found that it is possible to reduce selectively only the nitro group of the compound represented by formula (4) through hydrogenation in an alcohol solvent corresponding to R^{2b} in the presence of a platinum-based reduction catalyst. Thus, a compound represented by formula (5-1) can be obtained at high yield without use of iron powder, which presents problems in handling and reactivity, while removal of chlorine is prevented.
This reduction is performed in a solution of a starting compound represented by formula (4) or formula (5-2) in an alcohol corresponding to R^{2b} in the compound represented by formula (4) in the presence of a platinum-based reduction catalyst. Examples of the platinum-based reduction catalyst include platinum and Pt-S-carbon, with Pt-S-carbon being preferred. Pt-S-carbon is preferably added in an amount of 5% to 50%, more preferably 10% to 20%, by weight with respect to the weight of the compound represented by formula (4).

The hydrogen pressure is preferably normal pressure to 20 atm. Usually, the reaction is completed at normal pressure. The reaction temperature preferably falls within a range of 0°C to 50°C, more preferably room temperature to 50°C. The reaction time may be 1 hour to 24 hours. Usually, the reaction is completed within about 3 hours to about 10 hours.

After completion of reaction, the compound represented by formula (5-1) can be obtained as a hydrochloric acid salt in the following manner: hydrochloric acid, preferably HCl in ethanol, is added to the reaction mixture; the resultant mixture is stirred for a while at a temperature of 0°C to room temperature; and the crystals that have precipitated are collected through filtration. Alternatively, when crystals do not precipitate through the above process, crystals are allowed to precipitate through removal of the solvent, and then collected through filtration. The carboxylic acid represented by formula (6) can be obtained through collection of the crystals that have precipitated through filtration. Alternatively, in the case where crystals do not precipitate, the solvent is removed, and the crystals that have precipitated are collected through filtration.

Step c is a typical reaction of hydrolyzing an ester to a free carboxylic acid, and may be performed through a routine method for converting an alkoxycarbonyl group to a carboxylic acid moiety (see "Protective Groups in Organic Synthesis," edited by T. W. Greene and P. G. Wuts, John Wiley&Sons, Inc., New York, 1991).
Examples of the solvent employed in step c include alcohol solvents such as methanol and ethanol. Of these, methanol and ethanol are preferred.
The hydrolyzing reagent may be an acid or an alkali, such as a hydroxide of an alkali metal (e.g., sodium, potassium, lithium) or a hydroxide of an alkaline earth metal (e.g., magnesium, calcium). Of these, sodium hydroxide and potassium hydroxide are preferred.
The reaction temperature preferably falls within a range of room temperature to the boiling temperature of the solvent.
The reaction time may be 30 minutes to 5 hours. Usually, the reaction is completed within 1 hour to 2 hours.

### [Step d]

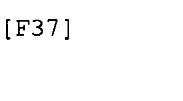

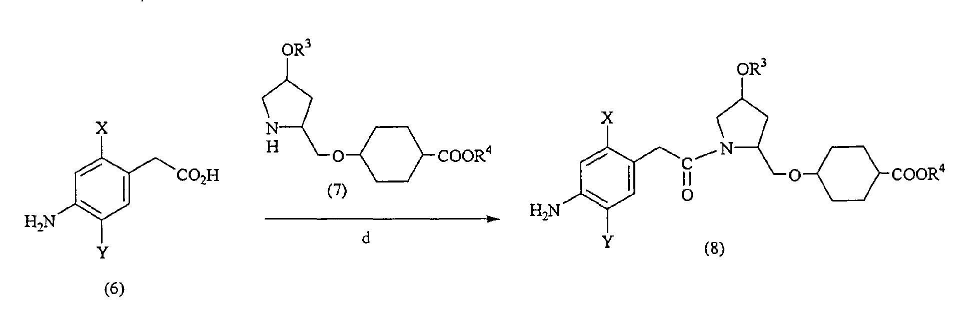

In step d, a carboxylic acid represented by formula (6) is condensed with a compound represented by formula (7). In the above reaction scheme, X, Y, R³, and R⁴ have the same meanings as defined above. Examples of the lower alkyl group represented by R³ include C1-C6 alkyl groups. The lower alkyl group is preferably methyl, ethyl, or a similar group, particularly preferably methyl. Examples of the lower alkyl group represented by R⁴ include C1-C6 alkyls. The aralkyl group is preferably phenyl-C₁₋₆ alkyl. Examples of the substituent which may be bound to the alkyl group or the aralkyl group include halogen atoms and C1-C6 alkoxy, and the number of the substituent may be 1 to 3. Examples of R⁴ include methyl, ethyl, tert-butyl, benzyl, and 4-methoxybenzyl, with methyl and ethyl being more preferred.

The compound represented by the above formula (7) may be produced through any of the methods described in WO2002/053534 and the specification of WO 2004/099136.

Step d may employ a common condensation reaction.
No particular limitation is imposed on the solvent employed, so long as the solvent does not adversely affect the reaction. Examples of the solvent include halogenated hydrocarbon solvents such as methylene chloride; hydrocarbon solvents such as toluene; ethereal solvents such as tetrahydrofuran; and aprotic polar solvents such as acetonitrile, N,N-dimethylformamide, and N-methyl-2-pyrrolidone. Of these, methylene chloride, acetonitrile, and N,N-dimethylformamide are preferred.

Step d may be performed in such a solvent, through use of a condensing agent such as 1-ethyl-3-(3-dimethylaminopropyl)carbodiimide hydrochloride, N,N-dicyclohexylcarbodiimide, N,N-carbonyldiimidazole, or a related compound. Preferably, the reaction is performed in N,N-dimethylformamide through use of 1-ethyl-3-(3-dimethylaminopropyl)carbodiimide hydrochloride or N,N-dicyclohexylcarbodiimide.

The reaction temperature may be a temperature of -20°C to the boiling temperature of the solvent, and is preferably 0°C to room temperature.

The reaction may be performed in the presence of an organic amine base such as triethylamine or N,N-dimethylaminopyridine, or an activated esterification reagent such as 1-hydroxybenzotriazole. The base or the reagent may be added in a catalytic amount to a stoichiometrically equivalent amount.

### [Step e]

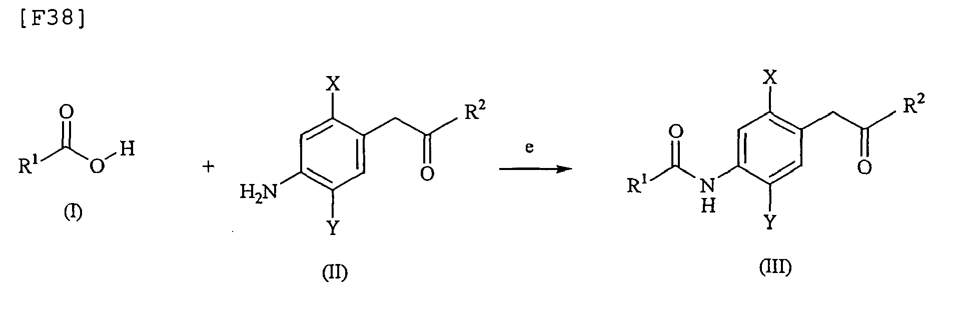

In step e, an amide linkage is formed in the compound represented by formula (II).
In formulas (I) and (III), R¹ represents an aryl group which may be substituted, or a heteroaryl group which may have substituent. R¹ is preferably a phenyl group which may be substituted, a benzofuranyl group which may be substituted, a thienyl group which may be substituted, a benzoisothiazolyl group which may be substituted, a benzothiophenyl group which may be substituted, an indolyl group which may be substituted, an indazolyl group which may be substituted, or an isoquinolinyl group which may be substituted. No particular limitation is imposed on the binding site of R¹. However, in the case of a benzofuranyl group which may be substituted, a thienyl group which may be substituted, a benzoisothiazolyl group which may be substituted, an indazolyl group which may be substituted, or an indolyl group which may be substituted, the group is preferably bound at the 3-position thereof. In the case of an isoquinolinyl group, the group is bound at the 1-position thereof.
Examples of the substituent include lower alkyl groups and halogen atoms. Of these, C1-C6 alkyls and halogen atoms are preferred, with methyl, chlorine atom, fluorine atom, and iodine atom being particularly preferred. No particular limitation is imposed on the number and the position of the substituent. When the heteroaryl group which may be substituted is an indolyl group which has been substituted by a methyl group, the methyl group is preferably substituted at the 1-position.

In formulas (II) and (III), R², X, and Y have the same meanings as defined above. Examples of the lower alkoxy group represented by R² include C1-C6 alkoxy groups. The aralkyloxy group is preferably phenyl-C₁₋₆ alkyloxy. Examples of the substituent which may be bound to the alkoxy group or the aralkyloxy group include halogen atoms and C1-C6 alkoxy groups, and the number of the substituent may be 1 to 3.

The compound represented by formula (II) includes compounds represented by formulas (5-1) and (8).

An amidation reaction similar to step e is described in WO2002/053534, in which a compound represented by formula (I) is dissolved in 1,2-dichloroethane, oxalyl chloride is added to the solution under cooling with ice, the mixture is stirred for a while and then concentrated to dryness, the formed crystals are dissolved in 1,2-dichloroethane, the solution is added to a solution of an ethyl ester compound (II) (wherein R² represents ethoxy) and triethylamine in 1,2-dichloroethane under cooling with ice, and the mixture is refluxed for about 10 hours.

The present inventors have found that the amidation reaction can be allowed to proceed quantitatively through reaction with a chlorinating agent under acidic conditions, without isolation of an acid chloride and without addition of a base.

Thus, a step of isolating an acid chloride can be eliminated, and a byproduct (i.e., dimer formed of carboxylic acid compounds (I)) can be suppressed to a minimum extent.
Examples of the reaction solvent which may be employed in this step include chlorinated solvents such as methylene chloride and 1,2-dichloroethane; hydrocarbon solvents such as toluene and benzene; ethereal solvents such as tetrahydrofuran; ester solvents such as ethyl acetate; and acetonitrile. The reaction solvent is preferably a chlorinated solvent such as methylene chloride or 1,2-dichloroethane, a hydrocarbon solvent such as toluene or benzene, or acetonitrile, particularly preferably 1,2-dichloroethane, toluene, or acetonitrile.
The chlorinating agent may be one which is usually employed for converting a carboxylic acid to an acid chloride (e.g., oxalyl chloride, thionyl chloride). Oxalyl chloride is particularly preferred.

In order to perform the reaction of step e under acidic conditions, a hydrochloric acid salt of a compound represented by formula (II) produced in step (b) or (d) may be reacted with a compound represented by formula (I) which has been treated with a chlorinating agent.

The formed acid chloride may be isolated through condensation of the reaction mixture. Alternatively, the compound (I) may be added to a reaction mixture without isolating the acid chloride from the reaction mixture. In this case, formation of an acid chloride is promoted through addition of N,N-dimethylformamide in an amount of 0.01% to 1% with respect to the weight of the compound represented by formula (5-1). After N,N-dimethylformamide has been added dropwise, the reaction may be performed at a temperature falling within a range of room temperature to the boiling temperature of the solvent, preferably 80°C to 130°C.
The reaction time is preferably 3 hours to 24 hours. Usually, the reaction is completed within about 3 hours to about 6 hours.

The compound represented by formula (I) may be synthesized by use of a commercially available compound as a starting material, if needed, through introduction of a substituent, in accordance with the method described in WO2002/053534.

### [Step f]

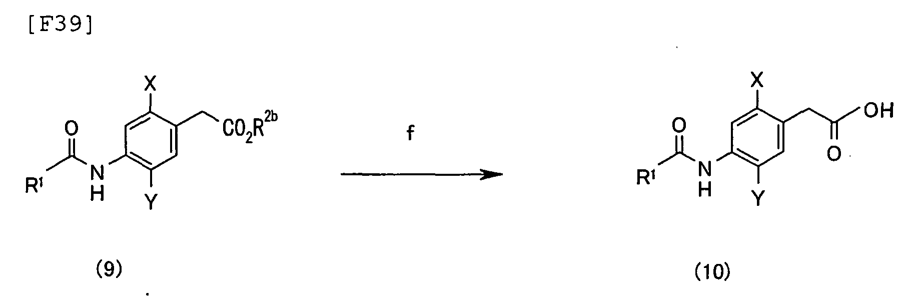

In step f, a compound represented by formula (9) is hydrolyzed to thereby yield a compound represented by formula (10). In the above reaction scheme, R¹, R^{2b}, X, and Y have the same meanings as defined above.

Step f is performed through a routine hydrolysis process, as described in relation to step c.

### [Step g]

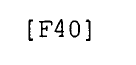

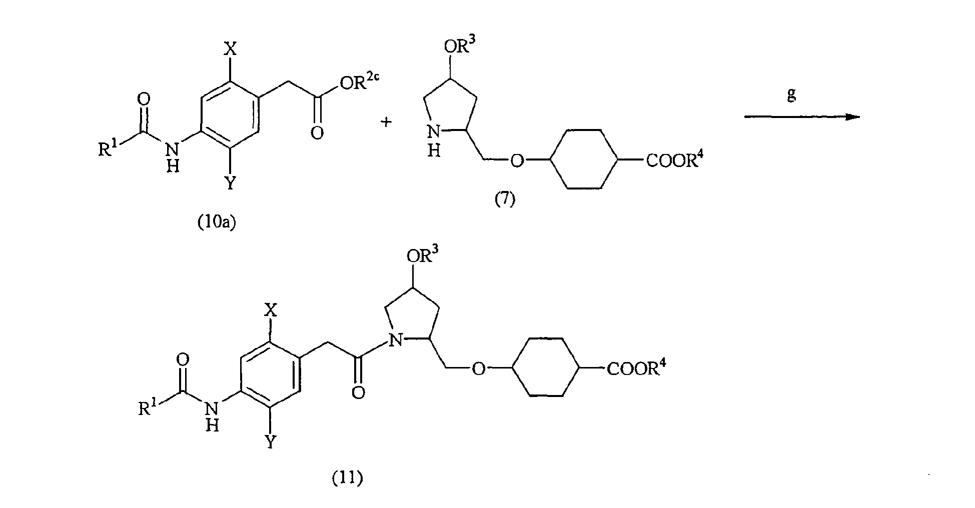

In step g, a compound represented by formula (10a) is condensed with a compound represented by formula (7), to thereby yield a compound represented by formula (11). The compound represented by formula (10a) is any of the compound represented by formula (9) and the compound represented by formula (10). In the above reaction scheme, R¹, R³, R⁴, X, and Y have the same meanings as defined above. R^{2c} represents a hydrogen atom or R^{2b}. When -OR^{2c} represents an ester, the ester is preferably an activated ester.

The condensation reaction may employ a known amidation process.

No particular limitation is imposed on the solvent employed in the reaction, so long as the solvent does not adversely affect the reaction. Examples of the solvent include halogenated hydrocarbon solvents such as methylene chloride; hydrocarbon solvents such as toluene; ethereal solvents such as tetrahydrofuran; and aprotic polar solvents such as acetonitrile, N,N-dimethylformamide, and N-methyl-2-pyrrolidone. Of these, methylene chloride, acetonitrile, and N,N-dimethylformamide are preferred.

The condensation reaction may be performed in the solvent through use of a condensing agent such as 1-ethyl-3-(3-dimethylaminopropyl)carbodiimide hydrochloride, N,N-dicyclohexylcarbodiimide, and N,N-carbonyldiimidazole. Preferably, the condensation reaction is performed in N,N-dimethylformamide through use of 1-ethyl-3-(3-dimethylaminopropyl)carbodiimide hydrochloride or N,N-dicyclohexylcarbodiimide.

The reaction temperature may fall within a range of - 20°C to the boiling temperature of the solvent, and is preferably 0°C to room temperature.

The condensation reaction may be performed in the presence of an organic amine base such as triethylamine or N,N-dimethylaminopyridine, or, alternatively, in the co-presence of an organic amine base and a catalytic amount to a stoichiometrically equivalent amount of an activated esterification reagent such as 1-hydroxybenzotriazole.

### [Step h]

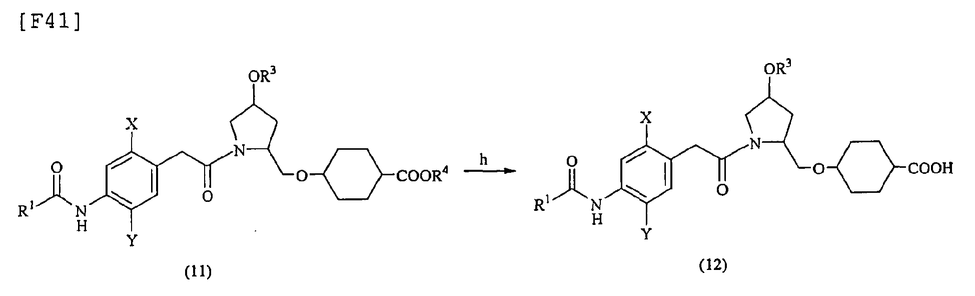

In step h, a compound represented by formula (11) is hydrolyzed to thereby yield a compound represented by formula (12). In the above reaction scheme, R¹, R³, R⁴, X, and Y have the same meanings as described above.

Step h is performed through a routine hydrolysis process, as described in relation to step c.

In the process of the present invention, the compound represented by formula (7) is preferably a compound represented by formula (7a):

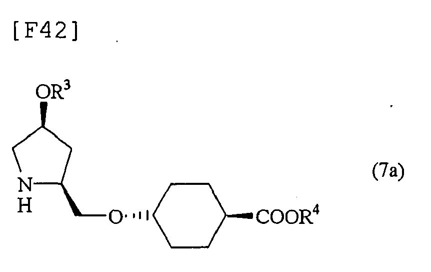

(wherein R³ and R⁴ have the same meanings as described above). More preferably, the compound represented by formula (7) is preferably a compound represented by formula (7a) wherein R³ represents a methyl group, and R⁴ represents an ethyl group.

The compounds produced in the steps of the process of the present invention may be isolated prior to being subjected to the respective subsequent step, or may be used without isolation in the respective subsequent step. The compound represented by formula (II); i.e., a compound represented by formula (5-1) or (8), is preferably used in the form of a hydrochloric acid salt in the subsequent step.

The compound represented by formula (12) may be formed into a salt such as a salt with an alkali metal such as sodium, potassium, or lithium; a salt with an alkaline earth metal such as magnesium or calcium; or a salt with an organic amine such as triethylamine, N-methylglucamine, N-benzylethanolamine, ethanolamine, tert-butylamine, tris(hydroxymethyl)aminomethane, or cyclohexylamine. The compound or the salt may be isolated in the form of a hydrate.

### [Step i]

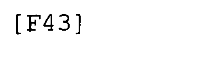

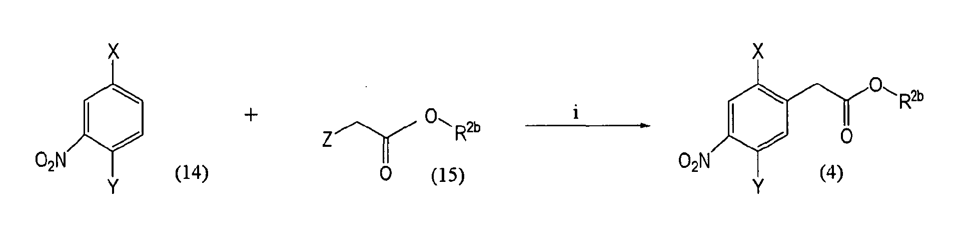

In step (i), a compound (14) is reacted with a compound (15) in the presence of a base in a solvent, whereby a - CH₂C(O)OR^{2b} group is introduced to the compound (14) at the para-position with respect to the nitro group (wherein R^{2b} has the same meaning as defined above).
As described above, the compound represented by formula (4) can be obtained through any of steps a and i.

In formulas (4) and (15), R^{2b} preferably represents a methyl group, an ethyl group, a tert-butyl group, or a phenyl group, more preferably a methyl group, an ethyl group, or a tert-butyl group, particularly preferably a tert-butyl group.
In formulas (4) and (14), X represents a hydrogen atom or a halogen atom, and Y represents a halogen atom or a lower alkoxy group. Examples of the lower alkoxy group include C1-C6 alkoxy groups. Preferably, each of X and Y independently represents a chlorine atom or a fluorine atom. More preferably, each of X and Y represents a chlorine atom. The compound represented by formula (14) may be purchased from, for example, Tokyo Chemical Industry Co., Ltd., or may be synthesized in accordance with the method described in Dunlop, Macrae, Tucker, J. Chem. Soc., 1672-1676 (1934).
In formula (15), Z represents a leaving group. No particular limitation is imposed on the leaving group, and a routinely used leaving group may be employed. Specific examples include halogen atoms, phenylthio, alkoxy, and amino. The leaving group is preferably a halogen atom or a phenylthio group, more preferably a chlorine atom or a bromine atom, particularly preferably a chlorine atom. The compound represented by formula (15) may be purchased from, for example, Tokyo Chemical Industry Co., Ltd.

The present inventors have found that a compound represented by formula (4) can be produced selectively, through reaction of a compound represented by formula (14) with a compound represented by formula (15). Actually, reaction of a compound represented by formula (14) with a compound represented by formula (15) also produced a byproduct compound represented by formula (16):

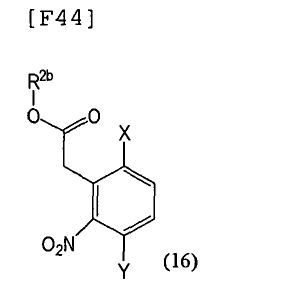

(wherein R^{2b}, X, and Y have the same meanings as defined above), in which a -CH₂C(O)OR^{2b} group is introduced at the ortho-position with respect to the nitro group. However, the ratio (I):(3) was found to be about 9:1. That is, the compound (4) was obtained at an unexpectedly high ratio. Before actually performing the above reaction, the present inventors could not estimate the selectivity, since they had considered that substitution might occur at the ortho- and/or para- positions with respect to the nitro group, and influence of the substituent X, which is adjacent to both of the ortho- and para-positions, could not be estimated.
In one preferred example, step i represents the following step j.

### [Step j]

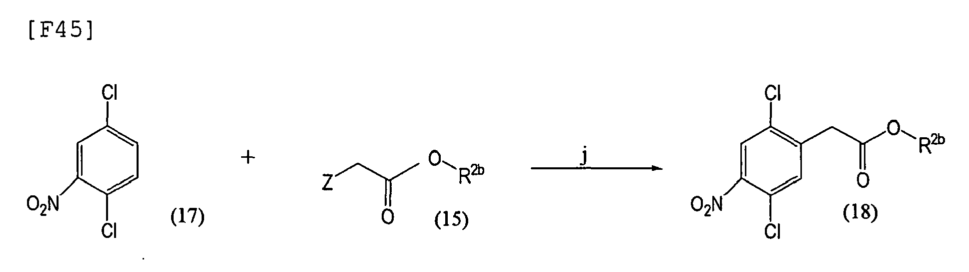

In step j, 2,5-dichloronitrobenzene (17) is reacted with a compound (15) in the presence of a base in a solvent, whereby a -CH₂C(O)OR^{2b} group (wherein R^{2b} has the same meaning as defined above) is introduced to the compound (17) at the para-position with respect to the nitro group. In the above reaction scheme, R^{2b} and Z have the same meanings as defined above.

2,5-Dichloronitrobenzene represented by formula (17) has been described in Can. J. Chem., 36, 238 (1958). A commercially available product of 2,5-dichloronitrobenzene may be employed, or 2,5-dichloronitrobenzene may be synthesized. For example, 2,5-dichloronitrobenzene may be purchased from Tokyo Chemical Industry Co., Ltd (Catalog No. D0387), or may be synthesized through the method described in Dunlop, Macrae, Tucker, J. Chem. Soc., 1672-1676 (1934).

Examples of the base employed in the above step (i) or (j) include, but are not limited to, sodium tert-butoxide, potassium tert-butoxide, sodium hydroxide, and potassium hydroxide. The base employed in the above step (i) or (j) is preferably sodium tert-butoxide or sodium hydroxide, more preferably sodium tert-butoxide. The amount of the base is preferably 2 to 10 eq., more preferably 2 to 3 eq., with respect to the amount of the compound (4). The base may be employed singly, or in combination of two or more species, so long as the reaction is not adversely affected.
Examples of the solvent employed in the above step (i) or (j) include, but are not limited to, amide solvents such as N,N-dimethylformamide and N,N-dimethylacetamide; ethereal solvents such as tetrahydrofuran, diethyl ether, dimethyl ether, and diisopropyl ether; ethyl acetate; and dimethyl sulfoxide (DMSO). Of these solvents, amide solvents and DMSO are preferred, with N,N-dimethylacetamide being more preferred. No particular limitation is imposed on the volume of the solvent, but the volume of the solvent is preferably 5 to 20 times, more preferably 10 to 15 times, the weight of the compound (4). The solvent may be employed singly, or in combination of two or more species, so long as the reaction is not adversely affected.
The reaction temperature of the steps (i) and (j) differs depending on the solvent employed. The reaction temperature is typically -20°C to 30°C, preferably -10°C to 0°C.
The reaction of the steps (i) and (j) is performed typically for about 10 minutes to about 24 hours, preferably for about 20 minutes to about 12 hours, more preferably for about 30 minutes to 3 hours, until the reaction is substantially completed.

Exemplary compounds which can be obtained through the process of the present invention are shown in Tables below.

**[Table 1]**

| No. | Compound | Structure | Analysis data |
|---|---|---|---|
| 1 | [5-Chloro-2-fluoro-4-[(6-fluoro-1-methyl-3-indo(ylcarbonyl)amino]ph enyl]acetic acid ethyl ester | 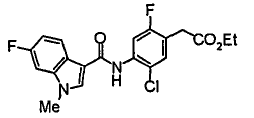 | ¹H-NMR (CDCl₃) δ: 1.28 (t, J=7.1 Hz, 3H), 3.62 (s, 2H), 3.84 (s, 3H), 4.19 (q, J=7.1 Hz, 2H), 7.07 - 7.12 (m, 2H), 7.34 (d, J=7.3 Hz, 1H), 7.74 (s, 1H), 8.10 (dd, J=9.1, 5.2 Hz, 1H), 8.19 (broad s, 1H), 8.49 (d, J=12.0 Hz, 1H). MS (ESI) m/z: 407 (M⁺+1) |
| 2 | [5-Chloro-2-fluoro-4-[(6-fluoro-1-methyl-3-indolylcarbonyl)amino]ph enyl]acetic acid | 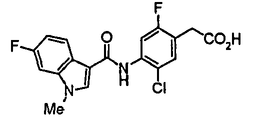 | ¹H-NMR (DMSO-d₆) δ: 3.65 (s, 2H), 3.86 (s, 3H), 7.06 (m, 1H), 7.44 (dd, J =10.3, 2.5 Hz, 1H), 7.54 (d, J=7.6 Hz, 1H), 7.69 (d, J=11.2 Hz, 1H), 8.12 (dd, J=8.8, 5.6 Hz, 1H), 8.32 (s, 1H), 9.38 (s, 1H), 12.58 (broad s, 1H). MS (ESI) m/z: 379 (M⁺+1) |
| 3 | {2-Fluoro-5-methoxy-4-[(1-methyl-1H-3-indolylcarbonyl)amino]ph enyl}acetic acid | 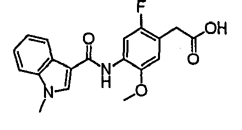 | ¹H-NMR (CDCl₃) δ: 3.60 (s, 2H), 3.90 (s, 6H), 7.05 (d, J=6.8 Hz, 1H), 7.22 (t, J=7.5 Hz, 1H), 7.30 (t, J=7.5 Hz, 1H), 7.58 (d, J=7.6 Hz, 1H), 7.95 (d, J=11.5 Hz, 1H), 8.12 (d, J=7.3 Hz, 1H), 8.31 (s, 1H), 8.80 (s, 1H). MS (ESI) m/z: 357 (M+1)⁺ |
| 4 | [3-Chloro-4-[(1-isoquinolinylcarbonyl)ami no]phenyl]acetic acid methyl ester | 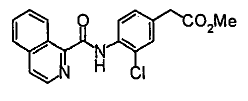 | ¹H-NMR (CDCl₃) δ: 3.62 (s, 2H), 3.72 (s, 3H), 7.29 (m, 1H), 7.40 (d, J= 1.9 Hz, 1H), 7.71 - 7.78 (m, 2H), 7.88 - 7.91 (m, 2H), 8.58 (d, J=5.4 Hz, 1H), 8.65 (d, J=8.3 Hz, 1H), 9.71 (m, 1H), 11.02 (m, 1H). MS (ESI) m/z: 355 (M⁺+1) |

**[Table 2]**

| No. | Compound | Structure | Analysis data |
|---|---|---|---|
| 5 | [3-Chloro-4-[(1-isoquinolinylcarbonyl)ami no]phenyl]acetic acid | 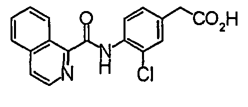 | ¹H-NMR (DMSO-d₆) δ: 3.63 (s, 2H), 7.33 (dd, J=8.3, 1.7 Hz, 1H), 7.51 (d, J=1.7 Hz, 1H), 7.81 (td, J=8.3, 1.7 Hz, 1H), 7.88 (td, J=8.3, 1.7 Hz, 1H), 8.12 (d, J=8.1 Hz, 1H), 8.16 (d, J=5.6 Hz, 1H), 8.24 (d, J=8.3 Hz, 1H), 8.67 (d, J=5.6 Hz, 1H), 9.34 (d, J=8.3 Hz, 1H), 10.85 (s, 1H), 12.48 (broad s, 1H). MS (ESI) m/z: 341 (M⁺+1) |
| 6 | [5-Chloro-2-fluoro-4-[(1-isoquinolinylcarbonyl)ami no]phenyl]acetic acid ethyl ester | 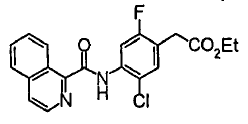 | ¹H-NMR (CDCl₃) δ: 1.28 (t, J=7.1 Hz, 3H), 3.64 (s, 2H), 4.19 (q, J=7.1 Hz, 2H), 7.36 (d, J=7.2 Hz, 1H), 7.72 - 7.78 (m, 2H), 7.88 - 7.91 (m, 2H), 8.57- 8.60 (m, 2H), 9.68 (m, 1H), 11.11 (broad s, 1H). MS (ESI) m/z: 387 (M⁺+1) |
| 7 | [5-Chloro-2-fluoro4-[(1-isoquinolinylcarbonyl)ami no]phenyl]acetic acid | 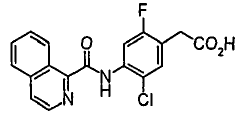 | ¹H-NMR (DMSO-d₆) δ: 3.66 (s, 2H), 7.62 (d, J=7.5 Hz, 1H), 7.82 (m, 1H), 7.88 (m, 1H), 8.12 (d, J=8.5 Hz, 1H), 8.18 (d, J=5.6 Hz, 1H), 8.23 (d, J=11.5 Hz, 1H), 8.67 (d, J=5.6 Hz, 1H), 9.38 (d, J=8.5 Hz, 1H), 10.98 (broad s, 1H), 12.59 (broad s, 1H). MS (ESI) m/z: 359 (M⁺+1) |
| 8 | {4-[(1-Benzofuran-3-ylcarbonyl)amino]-5-chloro-2-fluorophenyl}acetic acid | 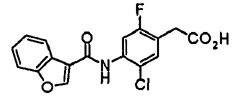 | ¹H-NMR (DMSO-d₆) δ: 3.67 (2H, s), 7.37-7.45 (2H, m), 7.57-7.61 (2H, m), 7.70 (1H, dd, J=7.3, 1.2 Hz), 8.07-8.10 (1H, m), 8.82 (1H, s), 9.99 (1H, s). MS (ESI) m/z: 348 [(M + H)⁺, ³⁵Cl], 350 [(M + H)⁺, ³⁷Cl] |

**[Table 3]**

| No. | Compound | Structure | Analysis data |
|---|---|---|---|
| 9 | [3-Chloro-4-[(1-methyl-3-indazolylcarbonyl)amino]p henyl]acetic acid methyl ester | 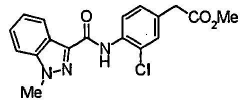 | ¹H-NMR (CDCl₃) δ: 3.61 (s, 2H), 3.72 (s, 3H), 4.18 (s, 3H), 7.24 (dd, J= 8.6, 2.0 Hz, 1H), 7.34 (m, 1H), 7.38 (d, J=2.0 Hz, 1H), 7.45 - 7.50 (m, 2H), 8.41 (d, J=8.0 Hz, 1H), 8.61 (d, J=8.6 Hz, 1H), 9.47 (broad s, 1H). MS (LCMS) m/z: 358 (M⁺+1) |
| 10 | [3-Chloro-4-[(1-methyl-3-indazolylcarbonyl)amino]p henyl]acetic acid | 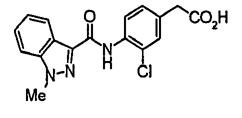 | ¹H-NMR (DMSO-d₆) δ: 3.62 (s, 2H), 4.21 (s, 3H), 7.28 (dd, J=8.3, 1.7 Hz, 1H), 7.35 (m, 1H), 7.47 (d, J=1.7 Hz, 1H), 7.53 (m, 1H), 7.80 (d, J=8.5 Hz, 1H), 8.11 (d, J=8.3 Hz, 1H), 8.21 (d, J=8.1 Hz, 1H), 9.69 (s, 1H), 12.49 (broad s, 1H). MS (LCMS) m/z: 344 (M⁺+1) |
| 11 | [5-Chloro-2-fluoro-4-[(1-methyl-3-indazolylcarbonyl)amino]p henyl]acetic acid ethyl ester | 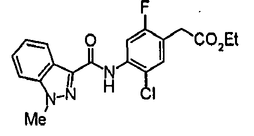 | ¹H-NMR (CDCl₃) δ: 1.28 (t, J=7.1 Hz, 3H), 3.62 (s, 2H), 4.17 (s, 3H), 4.18 (q, J=7.1 Hz, 2H), 7.33 - 7.39 (m, 2H), 7.44 - 7.50 (m, 2H), 8.38 (d, J= 8.3 Hz, 1H), 8.53 (d, J=11.9 Hz, 1H), 9.50 (broad s, 1H). MS (ESI) m/z: 390 (M⁺+1) |
| 12 | [5-Chloro-2-fluoro-4-[(1-methyl-3-indazolylcarbonyl)amino]p henyl]acetic acid | 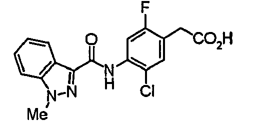 | ¹H-NMR (DMSO-d₆) δ: 3.65 (s, 2H), 4.21 (s, 3H), 7.37 (m, 1H), 7.53 (m, 1H), 7.59 (d, J=7.3 Hz, 1H), 7.80 (d, J=8.6 Hz, 1H), 8.13 (d, J=11.4 Hz, 1H), 8.21 (d, J=8.1 Hz, 1H), 9.68 (s, 1H), 12.58 (broad s, 1H). MS (ESI) m/z: 362 (M⁺+1). |

**[Table 4]**

| No. | Compound | Structure | Analysis data |
|---|---|---|---|
| 13 | [5-Chloro-2-fluoro-4-[(4-fluoro-1-methyl-3-indolylcarbonyl)amino]phe nyl]acetic acid ethyl ester | 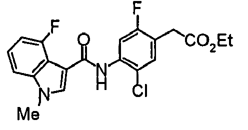 | ¹H-NMR (CDCl₃) δ: 1.27 (t, J=7.3 Hz, 3H), 3.61 (s, 2H), 3.87 (s, 3H), 4.18 (q, J=7.3 Hz, 2H), 7.02 (dd, J=12.2, 8.1 Hz, 1H), 7.20 (d, J=8.1 Hz, 1H), 7.27 (m, 1H), 7.32 (d, J=7.3 Hz, 1H), 8.02 (s, 1H), 8.48 (d, J=12.2 Hz, 1H), 9.26 (m, 1H). MS (ESI) m/z: 407 (M⁺+1) |
| 14 | [5-Chloro-2-fluoro-4-[(4-fluoro-1-methyl-3-indolylcarbonyl)amino]phe nyl]acetic acid | 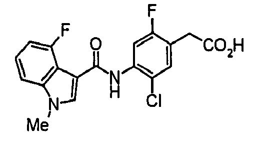 | ¹H-NMR (DMSO-d₆) δ: 3.64 (s, 2H), 3.90 (s, 3H), 7.07 (dd, J=12.5, 7.8 Hz, 1H), 7.29 (m, 1H), 7.44 (d, J=8.4 Hz, 1H), 7.55 (d, J=7.8 Hz, 1H), 8.11 (d, J=11.7 Hz, 1H), 8.25 (s, 1H), 9.30 (d, J=10.3 Hz, 1H), 12.54 (broad s, 1H). MS (LC/MS) m/z: 379 (M⁺+1) |
| 15 | [4-[(2-Indolylcarbonyl)amino]-3-methoxyphenyl]acetic acid | 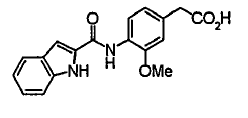 | ¹H-NMR (DMSO-d₆) δ: 3.57 (s, 2H), 3.86 (s, 3H), 6.87 (d, J=8.1 Hz, 1H), 7.01 (s, 1H), 7.06 (t, J=7.8 Hz, 1H), 7.21 (t, J=7.8 Hz, 1H), 7.32 (s, 1H), 7.45 (d, J=8.1 Hz, 1H), 7.64 (d, J=8.1 Hz, 1H), 7.74 (d, J=8.1 Hz, 1H), 9.32 (s, 1H), 11.72 (broad s, 1H). MS (ESI) m/z: 325 (M⁺+1) |
| 16 | [3-Bromo-4-[(1-isoquinolinylcarbonyl)amin o]phenyl]acetic acid ethyl ester | 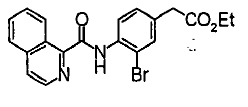 | ¹H-NMR (CDCl₃) δ: 1.28 (t, J=7.1 Hz, 3H), 3.60 (s, 2H), 4.17 (q, J=7.1 Hz, 2H), 7.32 (dd, J=8.5, 2.0 Hz, 1H), 7.57 (d, J=2.0 Hz, 1H), 7.71 - 7.78 (m, 2H), 7.88 - 7.91 (m, 2H), 8.59 (d, J=5.6 Hz, 1H), 8.63 (d, J=8.5 Hz, 1H), 9.71 (m, 1H), 11.01 (broad s, 1H). MS (ESI) m/z: 413 (M⁺+1), 415 (M⁺+3). |

**[Table 5]**

| No. | Compound | Structure | Analysis data |
|---|---|---|---|
| 17 | [3-Bromo-4-[(1-isoquinolinylcarbonyl)amin o]phenyl]acetic acid | 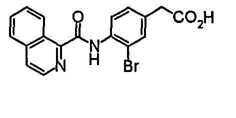 | ¹H-NMR (DMSO-d₆) δ: 3.63 (s, 2H), 7.36 (d, J=8.5 Hz, 1H), 7.66 (s, 1H), 7.81 (m, 1H), 7.88 (m, 1H), 8.12 (d, J=8.3 Hz, 1H), 8.17 (d, J=5.4 Hz, 1H), 8.21 (d, J=8.5 Hz, 1H), 8.66 (d, J=5.4 Hz, 1H), 9.36 (d, J=8.5 Hz, 1H), 10.84 (s, 1H). MS (ESI) m/z: 385 (M⁺+1), 387 (M⁺+3). |
| 18 | {4-[(1-Benzofuran-3-ylcarbonyl)amino]-2,5-dichlorophenyl}acetic acid | 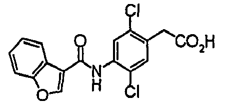 | ¹H-NMR (DMSO-d₆) δ: 3.76 (2H, s), 7.44 (2H, m), 7.68 (1H, s), 7.72 (1H, s), 7.81 (1H, s), 8.09 (1H, m), 8.83 (1H, m), 10.65 (1H, s), 12.61 (1H, br). |
| 19 | {5-Chloro-2-fluoro-4-[(1-methyl-3-indolylcarbonyl)amino]phe nyl}acetic acid | 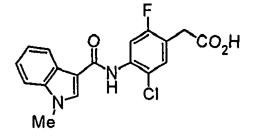 | ¹H-NMR (DMSO-d₆) δ: 3.65 (2H, s), 3.89 (3H, s), 7.21 and 7.28 (each 1H, each m), 7.56 (2H, m), 7.74 (1H, m), 8.15 (1H, m), 8.32 (1H, s), 9.32 (1H, s). |
| 20 | [3-Chloro-4-(2,6-dichlorobenzoylamino)phe nyl]acetic acid methyl ester | 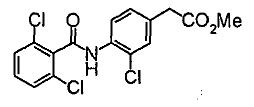 | ¹H-NMR (CDCl₃) δ: 3.61 (2H, s), 3.71 (3H, s), 7.25-7.39 (5H, m), 7.82 (1H, s), 8.46 (1H, d). |

**[Table 6]**

| No. | Compound | Structure | Analysis data |
|---|---|---|---|
| 21 | [3-Chloro-4-(2,6-dichlorobenzoylamino)phe nyl]acetic acid | 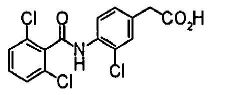 | ESI-MS m/z: 360 (M+3)⁺ |
| 22 | [5-Chloro-2-fluoro-4-(1-methyl-5-methoxy-3-indolylcarbonylamino)phe nyl]acetic acid ethyl ester | 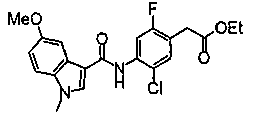 | ESI-MS m/z: 419 (M+H)⁺ |
| 23 | [5-Chloro-2-fluoro-4-(1-methyl-5-methoxy-3-indolylcarbonylamino)phe nyl]acetic acid | 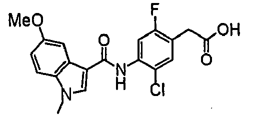 | ESI-MS m/z: 391 (M+H)⁺. |
| 24 | [5-Chloro-2-fluoro-4-[[(5-fluoro-1-methyl-3-indolyl)carbonyl]amino]ph enyl]acetic acid ethyl ester | 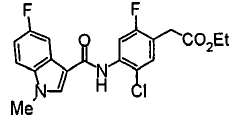 | ¹H-NMR (CDCl₃) δ: 1.27 (t, J=7.2 Hz, 3H), 3.61 (s, 2H), 3.87 (s, 3H), 4.18 (dd, J=7.2, 14.0 Hz, 2H), 7.08 (dt, J=2.0, 8.8 Hz, 1H), 7.29-7.34 (m, 2H), 7.5 (s, 1H), 7.83 (dd, J=2.4, 9.6 Hz, 1H), 8.11 (brs, 1H), 8.80 (d, J=12 Hz, 1H). MS (ESI) m/z: 407 (M⁺+1) |

**[Table 7]**

| No. | Compound | Structure | Analysis data |
|---|---|---|---|
| 25 | [5-Chloro-2-fluoro-4-[[(5-fluoro-1-methyl-3-indolyl)carbonyl]amino]ph enyl]acetic acid | 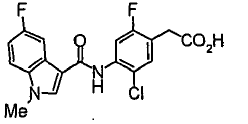 | MS (ESI m/z: 379 (M⁺+1). |
| 26 | [5-Chloro-2-fluoro-4-[[(1,2-dimethyl-1H-3-indolylcarbonyl)amino]phe nyl]acetic acid | 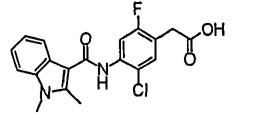 | ¹H-NMR (DMSO-d₆) δ: 2.70 (3H, s), 3.65 (2H, m), 3.77 (3H, s), 7.21 (2H, m), 7.58 (1H, d), 7.93 (1H, m), 7.99 (1H, d), 9.06 (1H, s), MS (ESI) m/z: 375 (M+1)⁺. |
| 27 | [3-Bromo-4-[(3-indolylcarbonyl)amino]phe nyl]acetic acid | 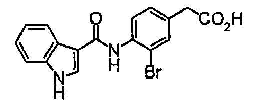 | ¹H-NMR (DMSO-d₆) δ: 3.62 (2H, s), 7.13 - 7.21 (2H, m), 7.29 (1H, dd), 7.47 (1H, d), 7.60 (1H, d), 7.63 (1H, d), 8.14 (1H, d), 8.27 (1H, d), 9.27 (1H, s). MS (ESI) m/z: 373 (M⁺+1), 375 (M⁺+3). |
| 28 | [2,5-Dichloro-4-[(1-methyl-3-indazolylcarbonyl)amino]p henyl]acetic acid ethyl | 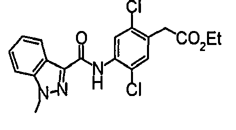 | ¹H-NMR (CDCl₃) δ: 1.28 (3H, t, J=7.1 Hz), 3.72 (2H, s), 4.18 (3H, s), 4.19 (2H, q, J=7.1 Hz), 7.35 (1H, m), 7.47 (1H, m), 8.40 (1H, d J=8.1 Hz), 8.82 (1H, s), 9.47 (1H, br). MS (ESI) m/z: 406 (M⁺+1), 408 (M⁺+3), 410 (M⁺+5). |

**[Table 8]**

| No. | Compound | Structure | Analysis data |
|---|---|---|---|
| 29 | [2,5-Dichloro-4-[(1-methyl-3-indazolylcarbonyl)amino]p henyl]acetic acid | 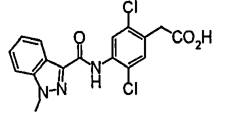 | ¹H-NMR (DMSO-d₆) δ: 3.27 (3H, s), 4.16 (2H, s), 7.30 (1H, m), 7.46 (2H, m), 7.73 (1H, m), 8.23 (1H, m), 9.67 (1H, s); MS (ESI) m/z: 400 (M⁺+1), 402 (M⁺+3), 404 (M⁺+5). |
| 30 | {3-Chloro-4-[(3-thienyl)carbonylamino]ph enyl}acetic acid ethyl ester | 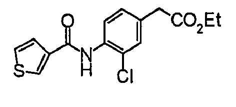 | ¹H-NMR (CDCl₃) δ: 1.29 (t, J=6.4 Hz, 3H), 3.60 (s, 2H), 4.18 (q, J=7.2, 14.4 Hz, 2H), 7.17 (brt, J=4.0 Hz, 1H), 7.24 (brd, J=10.4 Hz, 1H), 7.39 (brd, J=1.6 Hz, 1H), 7.60 (d, J=5.2 Hz, 1H), 7.68 (d, J=4.0 Hz, 1H), 8.29 (s, 1H), 8.46 (d, J=8.0 Hz, 1H). ESI-MS m/z: 324 (M⁺+1). |
| 31 | {3-Chloro-4-[(3-thienyl)carbonylamino]ph enyl}acetic acid | 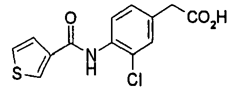 | ¹H-NMR (CDCl₃) δ: 3.61 (s, 2H), 7.21 (dd, J=4.0, 5.2 Hz, 1H) 7.25 dd, J=2.0, 8.4 Hz, 1H), 7.45 (d, J=1.6 Hz, 1H), 7.46 (d, J=8.4 Hz, 1H), 7.84 (dd, J=1.2, 5.2 Hz, 1H), 7.99 (brdd, J=2.8 Hz, 1H), 10.0 (s, 1H), 12.4 (s, 1H). ESI-MS m/z: 396 (M⁺+1). |
| 32 | {3-Chloro-4-[(1-methyl-3-indolylcarbonyl)amino]phe nyl}acetic acid ethyl ester | 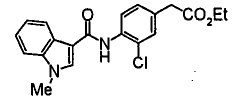 | ¹H-NMR (CDCl₃) δ: 1.27 (t, J=7.1 Hz, 3H), 3.58 (s, 2H), 3.89 (s, 3H), 4.17 (q, J=7.1 Hz, 2H), 7.24 (dd, J=8.3, 2.0 Hz, 1H), 7.32 - 7.43 (m, 4H), 7.81 (s, 1H), 8.16 (m, 1H), 8.28 (broad s, 1H), 8.59 (d, J=8.3 Hz, 1H). MS (ESI) m/z: 371 (M⁺+1), 373 (M⁺+3). |

**[Table 9]**

| No. | Compound | Structure | Analysis data |
|---|---|---|---|
| 33 | {3-Chloro-4-[(1-methyl-3-indolylcarbonyl)amino]phe nyl}acetic acid | 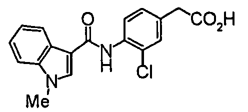 | ¹H-NMR (DMSO-d₆) δ: 3.61 (s, 2H), 3.89 (s, 3H), 7.17 - 7.28 (m, 3H), 7.43 (d, J=1.7 Hz, 1H), 7.53 (d, J=8.3 Hz, 1H), 7.69 (d, J=8.3 Hz, 1H), 8.14 (d, J=7.8 Hz, 1H), 8.26 (s, 1H), 9.26 (s, 1H). |
| 34 | {4-[(1-Benzo[b]thiophen-3-ylcarbonyl)amino]-5-chloro-2-fluorophenyl}acetic acid ethyl ester | 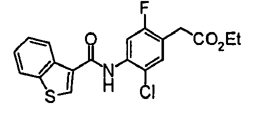 | ¹H-NMR (CDCl₃) δ: 1.28 (3H, t, J=7.1 Hz), 3.64 (2H, s), 4.20 (2H, q, J=7.2 Hz), 7.36 (1H, d, J=7.3 Hz), 7.44-7.55 (2H, m), 7.92 (1H, d, J=8.3 Hz), 8.09 (1H, s), 8.34 (1H, br s), 8.47-8.50 (2H, m). MS (ESI) m/z: 392 [(M + H)⁺, ³⁵Cl], 394 [(M + H)⁺, ³⁷Cl]. |
| 35 | {4-[(1-benzo[b]thiophen-3-ylcarbonyl)amino]-5-chloro-2-fluorophenyl}acetic acid | 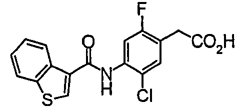 | ¹H-NMR (DMSO-d₆) δ: 3.67 (2H, s), 7.44-7.60 (4H, m), 8.08 (1H, d, J=7.8 Hz), 8.45 (1H, d, J=8.1 Hz), 8.65 (1H, d, J=1.2 Hz), 10.10 (1H, s). MS (ESI) m/z: 364 [(M + H)⁺, ³⁵Cl], 366 [(M + H)⁺, ³⁷Cl] |
| 36 | {4-[(1-Benzo[b]thiophen-3-ylcarbonyl)amino]-2,5-dichlorophenyl}acetic acid ethyl ester | 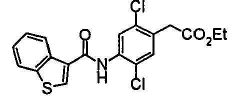 | ¹H-NMR (CDCl₃) δ: 1.28 (3H, t, J=7.2 Hz), 3.73 (2H, s), 4.20 (2H, q, J=7.1 Hz), 7.39 (1H, s), 7.44-7.55 (2H, m), 7.91-7.94 (1H, m), 8.09 (1H, s), 8.29 (1H, br s), 8.47-8.50 (1H, m), 8.75 (1H, s). MS (ESI) m/z: 408 [(M + H)⁺, ³⁵Cl + ³⁵Cl], 410 [(M + H)⁺, ³⁷Cl + ³⁵Cl, ³⁵Cl + ³⁷Cl], 412 [(M + H)⁺, ³⁷Cl + ³⁷Cl]. |

**[Table 10]**

| No. | Compound | Structure | Analysis data |
|---|---|---|---|
| 37 | {4-[(1-Benzo[b]thiophen-3-ylcarbonyl)amino]-2,5-dichlorophenyl}acetic acid | 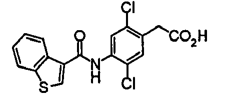 | ¹H-NMR (DMSO-d₆) δ: 3.78 (2H, s), 7.48 (2H, m), 7.68 (1H, s), 7.80 (1H, s), 8.10 (1H, s), 8.45 (1H, m), 8.65 (1H, s), 10.17 (1H, s), 12.61 (1H, br). |
| 38 | {4-[(Benzo[d]isothiazole-3-carbonyl)amino]-3-chlorophenyl}acetic acid | 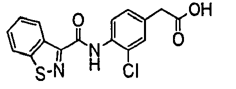 | ESI-MS m/z: 347 (M+1)⁺; ¹H-NMR (DMSO-d₆) δ: 3.62 (s, 2H), 7.30 (d, J=8.1 Hz, 1H), 7.50 (s, 1H), 7.64 (d, J=8.1 Hz, 1H), 7.70 (d, J=8.1 Hz, 1H), 7.95 (d, J=8.3 Hz, 1H), 8.36 (d, J=8.1 Hz, 1H), 8.80 (d, J=8.1 Hz, 1H), 10.27 (s, 1H). |
| 39 | {5-Chloro-2-fluoro-4-[(4-fluorobenzo[d]isothiazole -3-carbonyl)amino]phenyl}a cetic acid | 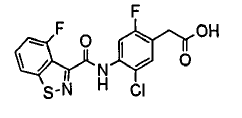 | ¹H-NMR (DMSO-d₆) δ: 1.28 (t, J = 7.2 Hz, 3H), 3.64 (s, 2H), 4.19 (q, J = 7.2 Hz, 2H), 7.34 (dt, J = 2.2, 9.1 Hz, 1H), 7.37 (d, J = 7.1 Hz, 1H), 7.64 (dd, J = 2.2, 8.3 Hz, 1H), 8.48 (d, J = 11.5 Hz, 1H), 9.00 (dd, J = 5.1, 9.0 Hz, 1H), 9.92 (s, 1H). |

**[Table 11]**

| No. | Compound | Structure | Structure analysis data |
|---|---|---|---|
| 40 | (2,5-Dichloro-4-{[(7-fluoro-1H-indol-3-yl)carbonyl]amino}phen yl)acetic acid ethyl ester | 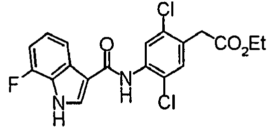 | ¹H-NMR (CDCl₃) δ: 1.29 (3H, t, J = 7.1 Hz), 3.73 (2H, s), 4.20 (2H, q, J = 7.1 Hz), 7.04 (1H, dd, J = 10.7 and 8.0 Hz), 7.20 - 7.30 (1H, m), 7.36 (1H, s), 7.89 (1H, d, J = 8.0 Hz), 7.93 (1H, d, J = 3.2 Hz), 8.25 (1H, broad s), 8.77 (1H, s), 8.99 (1H, broad s). MS (FAB) m/z: 409 [(M⁺+1), ³⁵Cl + ³⁵Cl], 411 [(M⁺+1), ³⁵Cl + ³⁷Cl], 413 [(M⁺+1), ³⁷Cl + ³⁷Cl]. HRMS (FAB) Calcd for C₁₉H₁₆Cl₂FN₂O₃: 409.0522, Found: 409.0512. |
| 41 | (2,5-Dichloro-4-{[(7-fluoro-1H-indol-3-yl)carbonyl]amino}phen yl)acetic acid | 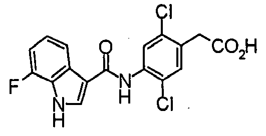 | ¹H-NMR (DMSO-d₆) δ: 3.73 (2H, s), 7.04 (1H, ddd, J=10.7, 8.0 and 0.5 Hz), 7.12 (1H, dt, J=8.0 and 4.9 Hz), 7.63 (1H, s), 7.84 (1H, s), 7.94 (1H, d, J=8.0 Hz), 8.37 (1H, d, J=2.9 Hz), 9.55 (1H, s), 12.34 (1H, broad s), 12.58 (1H, broad s). MS (FAB) m/z: 381 [(M⁺+1), ³⁵Cl + ³⁵Cl], 383 [(M⁺+1), ³⁵Cl + ³⁷Cl], 385 [(M⁺+1), ³⁷Cl + ³⁷Cl]. |

### Examples

The present invention will next be described in detail by way of Examples, which should not be construed as limiting the invention thereto.

### Example 1

### (2,5-Dichloro-4-nitrophenyl)acetic acid ethyl ester

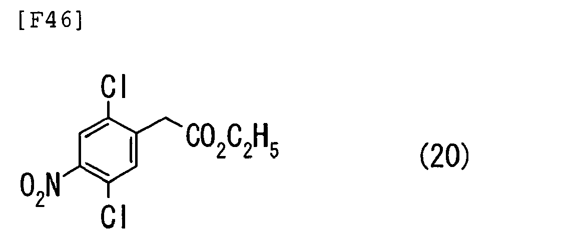

In a flask, ethanol (250 mL) and concentrated sulfuric acid (2.5 mL) were added to 2,5-dichloro-4-nitrophenylacetic acid (50.00 g), and the mixture was refluxed for 6 hours. After completion of reaction had been confirmed through HPLC, activated carbon was added to the mixture, and the resultant mixture was stirred for 30 minutes, followed by filtration. Water (400 mL) was gradually poured into the filtrate, and the mixture was cooled and then stirred for a while. The crystals that precipitated were collected through filtration, washed with water, and then dried under reduced pressure at 50°C, to thereby yield the compound represented by formula (20) (50.15 g, 90.2%).
¹H-NMR(CDCl₃)δ: 1.28(t,J=7.1Hz,3H), 3.80(s,2H), 4.21(q,J=7.1Hz,2H), 7.53(s,1H), 7.97(s,1H)

### Example 2

### (4-Amino-2,5-dichlorophenyl)acetic acid ethyl ester hydrochloride

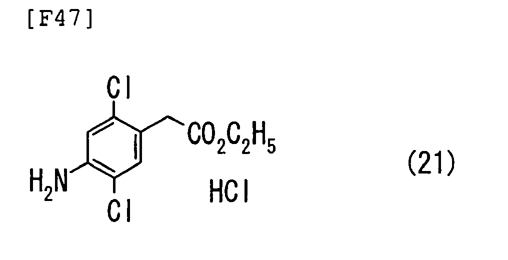

Ethanol (350 mL) and 3% Pt-S-carbon (50% WET) (7.5 g) were added to the compound obtained in Example 1 (50.00 g), and the mixture was stirred for 7 hours under hydrogen flow at room temperature. After completion of reaction had been confirmed through HPLC, the reaction mixture was subjected to filtration. 1N HCl-ethanol was added to the filtrate, and the solvent was removed. Acetonitrile (150 mL) was added to the residue. The mixture was cooled, and the crystals that precipitated were collected through filtration, washed with acetonitrile, and dried, to thereby yield the hydrochloric acid salt represented by formula (21) (43.43 g, 84.9%). ¹H-NMR(DMSO)δ: 1.17(t,J=7.1Hz,3H), 3.60(s,2H), 4.08(q,J=7.1Hz,2H), 6.10(br-s,3H), 6.89(s,1H), 7.26(s,1H)

### Example 3

### (2,5-Dichloro-4-nitrophenyl)acetic acid

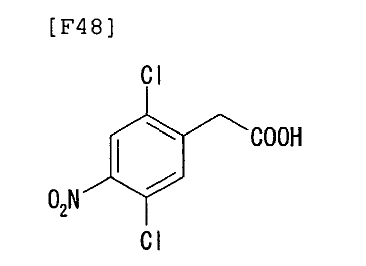

(1) 1N HCl/acetic acid (4 mL) was added to and dissolved in (2,5-dichloro-nitrophenyl)acetic acid tert-butyl ester (400 mg) at an internal temperature of 25°C or lower, and the mixture was stirred for 4 hours. After completion of reaction had been confirmed through HPLC, water (30 mL) was added to the reaction mixture under cooling with ice, and the resulting mixture was extracted three times with ethyl acetate (10 mL). The combined extract was dried over magnesium sulfate and then subjected to filtration. The solvent was removed under reduced pressure, to thereby yield the title compound (294 mg, 90%) as a yellow powder.
   ¹H-NMR(DMSO-d6, 400MHz)δ: 3.86(s,2H,Ph-CH2-), 7.96(s,1H,Ph), 8.30(s,1H,Ph), 12.81(br,1H,0H)

(2) Ethanol (20 mL) was added to and suspended in (2,5-dichloro-4-nitrophenyl)acetic acid ethyl ester (2.0 g) at room temperature. 1N aqueous sodium hydroxide (10 mL, 1.4 eq.) was added to the suspension, and the mixture was heated to 70°C and then stirred for 2 hours. After completion of reaction had been confirmed through HPLC, acetic acid (0.6 g, 1.4 eq.) and water (10 mL) were added to the reaction mixture at room temperature. The resulting mixture was extracted twice with ethyl acetate (20 mL). The combined extract was washed with 2N hydrochloric acid and saturated brine. The mixture was dried over magnesium sulfate and then filtered. The solvent of the filtrate was removed under reduced pressure, to thereby yield the title compound (1.8 g, 100%) as a yellow powder.

### Example 4

### (2,5-Dichloro-4-aminophenyl)acetic acid

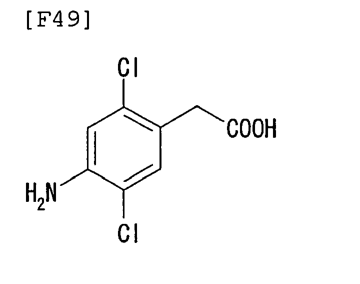

Ethanol (7 mL) and 3% Pt-S-carbon (50% Wet, 150 mg) were added to (2,5-dichloro-4-nitrophenyl)acetic acid (1.0 g), and the mixture was stirred for 5 hours under hydrogen flow at room temperature. After completion of reaction had been confirmed through HPLC, the reaction mixture was subjected to filtration, and the solvent was removed under reduced pressure, to thereby yield the title compound (0.84 g, 95%) as a yellowish brown solid.
¹H-NMR(DMSO-d6, 400MHz)δ: 3.51(s,2H,Ph-CH2-), 5.53(s,1H,NH2), 6.84(s,1H,Ph), 7.23(s,1H,Ph), 12.33(br,1H,OH)

### Example 5

### {2,5-Dichloro-4-[(1-methylindol-3-yl)carboxamido]phenyl}acetic acid ethyl ester

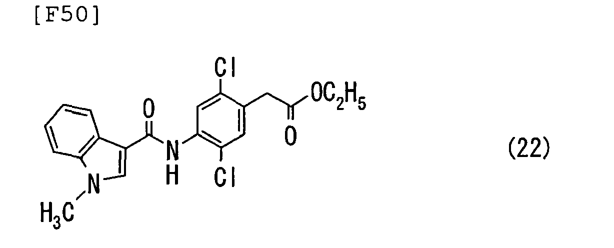

A compound represented by formula (I) wherein R¹ represents a 1-methylindol-3-yl group (10 g) and toluene (150 mL) were added to a flask, and, at room temperature, (COCl)₂ (5.6 mL) was added dropwise to the mixture. Subsequently, DMF (20 µL) was added to the mixture, and the resultant mixture was stirred for 1 hour at room temperature by means of a mechanical stirrer. The compound obtained in Example 2 (16.6 g) was added to the reaction mixture, and the resultant mixture was refluxed for 4 hours at an external temperature of 100°C to 120°C. After completion of reaction had been confirmed through HPLC, the reaction mixture was cooled, diisopropyl ether (IPE) (50 mL) was added to the reaction mixture, and the resultant mixture was stirred for a while. The crystals that precipitated were collected through filtration, washed with IPE, and dried, to thereby yield the title compound represented by formula (22) (20.7 g, 89.5%).
¹H-NMR(CDCl₃)δ: 1.28(t,J=7.1Hz,3H), 3.72(s,2H), 3.89(s,3H), 4.20(q,J=7.1Hz,2H), 7.32-7.44(m,4H), 7.80(s,1H), 8.10- 8.16(m,1H), 8.25(s,1H), 8.81(s,1H)

### Example 6

### {2,5-Dichloro-4-[(1-methylindol-3-yl)carboxamido]phenyl}acetic acid

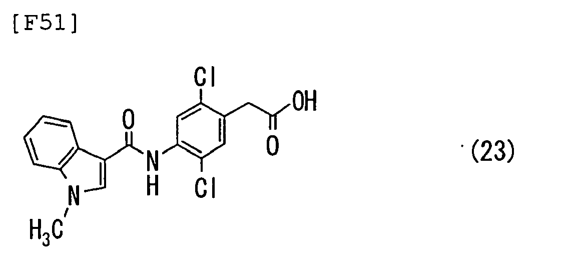

The compound obtained in Example 5 (20 g), methanol (300 mL), and 0.5mol/L sodium hydroxide (200 mL) were added to a flask, and the mixture was refluxed for 1.5 hours. After completion of reaction had been confirmed through HPLC, acetic acid (14.1 mL) was added to the reaction mixture at an internal temperature of 70°C, and the resultant mixture was stirred for a while until the mixture was cooled to room temperature. The crystals that precipitated were collected through filtration, washed with water, and dried, to thereby yield the compound represented by formula (23) (16.75 g, 90.0%).
¹H-NMR (DMSO)δ: 3.74(s,2H), 3.89(s,3H), 7.19-7.31(m,2H), 7.56(d,J=7.1Hz,1H), 7.64(s,1H), 7.93(s,1H,8.15(d,J=7.1Hz,1H), 8.31(s,1H), 9.39(s,1H), 12.59(br-s,1H)

### Example 7

### trans-4-((2S,4S)-1-{2,5-Dichloro-4-[(1-methylindol-3-yl)carboxamido]phenyl}acetyl-4-methoxypyrrolidin-2-yl)methoxycyclohexane-1-carboxylic acid ethyl ester

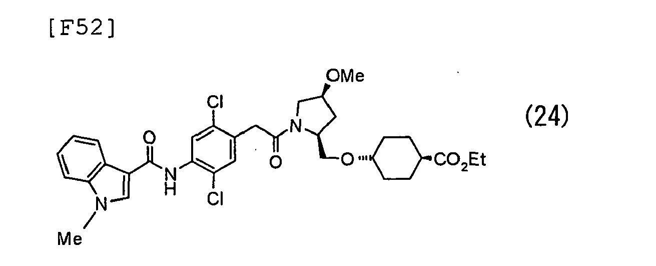

The compound obtained in Example 6 (25.00 g), trans-4-[(2S,4S)-4-methoxypyrrolidin-2-yl]methoxycyclohexane-1-carboxylic acid ethyl ester (35.00 g), 1-hydroxybenzotriazole (10.75 g), and acetonitrile (250 mL) were added to a flask, and triethylamine (20.3 mL) was added to the mixture under stirring at room temperature. After the mixture had become a solution, 1-ethyl-3-(3-dimethylaminopropyl)carbodiimide hydrochloride (17.79 g) was added to the mixture, and the resultant mixture was stirred for 5 hours at room temperature by means of a mechanical stirrer. After completion of reaction had been confirmed through HPLC, water (200 mL) was added to the reaction mixture. The resultant mixture was stirred for a while, and the crystals that precipitated were collected through filtration, sequentially washed with water (100 mL), water:acetonitrile (1:1) (100 mL), and water:isopropyl alcohol (1:1) (100 mL), and dried under reduced pressure at 50°C, to thereby yield the compound represented by formula (24) (40.27 g, 94.3%) [isomer 0.88%]. ¹H-NMR(CDCl₃)δ: 1.14-1.33(m,6H), 1.36-1.55(m,2H), 1.92-2.14(m,4H), 2.15-2.43(m,2H), 3.18-3.35(m, including 2s,at δ 3.30,3.33,total 8H), 3.44-3.58(m,2H), 3.62-4.03(series of m, including s at δ 3.86,total 8H), 4.09(q,J=6.8Hz,2H), 4.25(m,1H), 7.19-7.45(series of m, total 4H), 7.78(s,1H), 8.13(m,1H), 8.22(brd,J=3.2Hz,1H), 8.77(d,J=7.2Hz,1H).

### Example 8

### trans-4-((2S,4S)-1-{2,5-Dichloro-4-[(1-methylindol-3-yl)carboxamido]phenyl}acetyl-4-methoxypyrrolidin-2-yl)methoxycyclohexane-1-carboxylic acid sodium salt pentahydrate

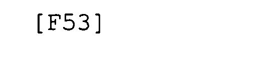

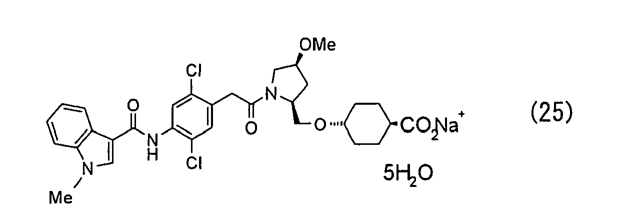

(1) To a flask equipped with a mechanical stirrer, the compound obtained in Example 7 (20.00 g), isopropyl alcohol (200 mL), and 1N NaOH (34.1 mL) were added, and the mixture was refluxed for 2.5 hours (internal temperature: 62 to 72°C). After completion of reaction had been confirmed through HPLC, the mixture was allowed to cool to 60°C, and isopropyl alcohol (100 mL) was added thereto. The resultant mixture was stirred for a while until the mixture was cooled to room temperature. The crystals that precipitated were collected through filtration, washed with isopropyl alcohol, and dried with air, to thereby yield crude crystals of the compound represented by formula (25) (19.31 g) (the crude crystals were in the form of a hydrate of an Na salt, but the number of hydrated water molecules remained undetermined, since the crude crystals were slightly amorphous).
(2) The crude crystals (15.00 g) and 50% aqueous acetone (90 mL) were added to a flask, and the mixture was heated to 30 to 40°C for dissolution of the crude crystals. Activated carbon (0.75 g) was added to the resultant solution, and the mixture was stirred for 30 minutes and then subjected to filtration, followed by washing with 10% aqueous acetone (10 mL). Acetone (360 mL) was added to the filtrate, and the mixture was gently stirred for 20 hours at room temperature by means of a mechanical stirrer. The crystals that precipitated were collected through filtration, washed with 10% aqueous acetone, and dried for 20 hours at 50°C (12.71 g). Moisture conditioning was performed at room temperature for 2 days, to thereby yield the compound represented by formula (5) (14.18 g, 80.8% as calculated with respect to the compound represented by formula (24)). The crystal form of the compound was determined through X-ray powder diffraction. ¹H-NMR(DMSO-d6)δ: 1.09-1.43(m,4H), 1.80-2.22(m,7H), 3.10-4.30(series of m, including s at δ 3.89,total 12H), 7.21(dd,J=7.6,7.6Hz,1H), 7.28(dd,J=7.6,7.6Hz,1H), 7.49and7.52(2S,total 1H), 7.56(d,J=7.6Hz,1H), 7.89and7.90(2S,total 1H), 8.15(d,J=7.6Hz,1H), 8.30(s,1H), 9.37(s,1H)

### Example 9

### trans-4-[(2S,4S)-1-(4-Amino-2,5-dichlorophenyl)acetyl-4-methoxypyrrolidin-2-yl]methoxycyclohexane-1-carboxylic acid ethyl ester

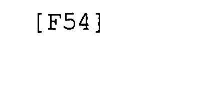

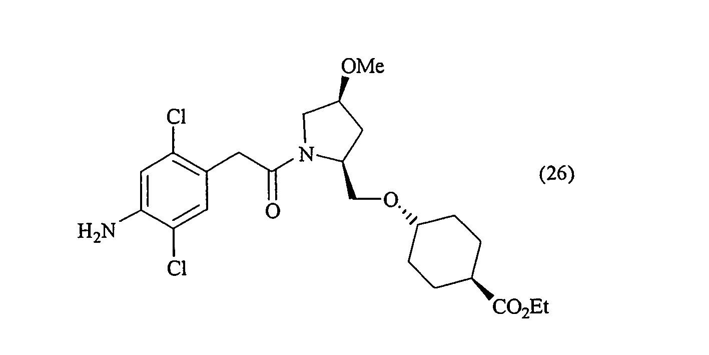

trans-4-[(2S,4S)-4-Methoxypyrrolidin-2-yl]methoxycyclohexane-1-carboxylic acid ethyl ester (1.21 g, 5.45 mmol), (4-amino-2,5-dichlorophenyl)acetic acid (1.2 g, 5.45 mmol), 4-dimethylaminopyridine (700 mg, 5.73 mmol), and a catalytic amount of 1-hydroxybenzotriazole were added to DMF (50 mL). Under stirring at room temperature, 1-ethyl-3-(3-dimethylaminopropyl)carbodiimide hydrochloride (1.25 g, 6.54 mmol) was added to the mixture. The reaction mixture was stirred for 15 hours at room temperature. The reaction mixture was poured into ice-water (100 mL), and the resultant mixture was extracted with ethyl acetate. The extract was washed with saturated brine (twice), and then dried over anhydrous sodium sulfate. The solvent was removed under reduced pressure. The residue was purified through silica gel column chromatography, and, from a fraction obtained through use of chloroform:ethyl acetate (9:1 to 4:1, v/v), there was obtained trans-4-[(2S,4S)-1-(4-amino-2,5-dichlorophenyl)acetyl-4-methoxypyrrolidin-2-yl]methoxycyclohexane-1-carboxylic acid ethyl ester (formula (26)) (2.48 g, 94%) as a colorless solid.
Melting point (uncorrected): 113-118°C
IR(ATR)cm⁻¹: 3464,3303,3182,1726,1633.
¹H-NMR(CDCl₃)δ: 1.22-1.26(5H,m), 1.42-1.48(2H,m), 1.95-1.99(5H,m), 2.03-2.08(2H,m), 3.22-4.12(14H,m), 6.78(1H,m), 7.18(1H,m).
MS(ESI); m/z: 488(M⁺-1).
Anal.;
Calcd for C₂₃H₃₂C₁₂N₂O₅: C,56.88; H,6.62; N,5.75.
Found: C,56.57; H,6.62; N,5.64.

### Example 10

### trans-4-((2S,4S)-1-{2,5-Dichloro-4-[(1-methylindol-3-yl)carboxamido]phenyl}acetyl-4-methoxypyrrolidin-2-yl)methoxycyclohexane-1-carboxylic acid ethyl ester

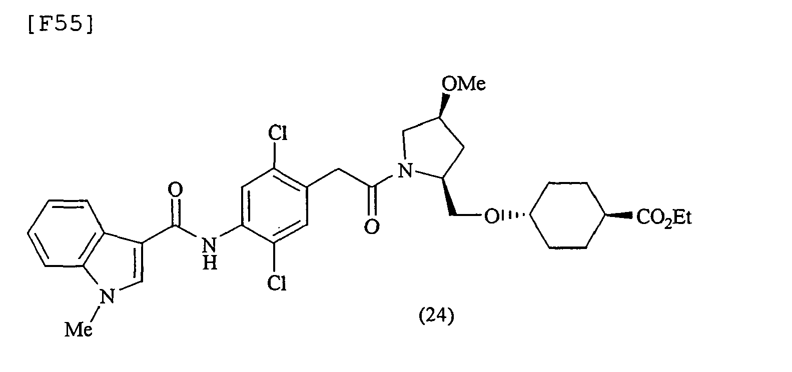

While 1-methylindole-3-carboxylic acid (150 mg, 0.86 mmol) and 1,2-dichloroethane (3 mL) were stirred under cooling in an ice-water bath, oxalyl chloride (0.095 mL, 1.07 mmol) was added to the mixture, and the resultant mixture was stirred for 1 hour at the same temperature. The reaction mixture was completely dried under reduced pressure. The crystals that precipitated were dissolved in 1,2-dichloroethane (3 mL), and the solution was added to trans-4-[(2S,4S)-1-(4-amino-2,5-dichlorophenyl)acetyl-4-methoxypyrrolidin-2-yl]methoxycyclohexane-1-carboxylic acid ethyl ester (formula (26)) (348 mg, 0.714 mmol) in 1,2-dichloroethane (15 mL) under stirring and cooling. After completion of addition, the reaction mixture was refluxed for 10 hours under stirring. The reaction mixture was cooled, washed with water, and dried over anhydrous sodium sulfate. The solvent was removed under reduced pressure. The residue was purified through silica gel column chromatography, and, from a fraction obtained through use of chloroform:ethyl acetate (9:1 to 3:1, v/v), the title compound (24) was obtained as a crystalline powder (350 mg, 76%). Spectrum data obtained from the compound produced through this process were identified with those obtained through the above process.

### Example 11

### trans-4-((2S,4S)-1-{2,5-Dichloro-4-[5-iodo-(1-methylindol-3-yl)carboxamido]phenyl}acetyl-4-methoxypyrrolidin-2-yl)methoxycyclohexane-1-carboxylic acid ethyl ester

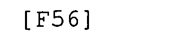

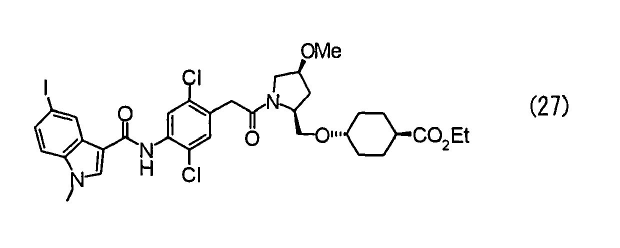

Oxalyl chloride (0.135 mL, 1.54 mmol) was added to 5-iodoindole-3-carboxylic acid (370 mg, 1.23 mmol) in 1,2-dichloroethane (10 mL) under stirring at 0°C. The reaction mixture was stirred for 1 hour at 0°C and for 5 hours at room temperature. The solvent of the reaction mixture was removed under reduced pressure. The obtained acid chloride was employed in the next step.
A solution of the above acid chloride in 1,2-dichloroethane (5 mL) was added dropwise to trans-4-[(2S,4S)-1-(4-amino-2,5-dichlorophenyl)acetyl-4-methoxypyrrolidin-2-yl]methoxycyclohexane-1-carboxylic acid ethyl ester (500 mg, 1.03 mmol) in 1,2-dichloroethane (30 mL) under stirring at 0°C. The reaction mixture was stirred for 16 hours under reflux conditions. The reaction mixture was cooled to room temperature, and water (20 mL) was added thereto. The resultant mixture was extracted with chloroform. The extract was washed with saturated brine, dried over anhydrous sodium sulfate, and then concentrated to dryness under reduced pressure. The residue was purified through silica gel column chromatography, and, from a fraction obtained through use of chloroform-ethyl acetate (2:1, v/v), the title compound (27) was obtained as an amorphous form (650 mg, 82%).
¹H-NMR(CDCl₃)δ: 1.20-1.30(5H,m), 1.45-1.50(2H,m), 1.95-2.35(10H,m and s), 3.25(1H,m), 3.31 and 3.34(3H,each s), 3.45-4.32(11H,series of m), 7.16(1H,m), 7.41(1H,m), 7.60(1H,m), 7.69(1H,m), 8.09(1H,m), 8.59(1H,m), 8.74(1H,m).

### Example 12

### trans-4-((2S,4S)-1-{2,5-Dichloro-4-[(1-methyl-d3-indol-3-yl)carboxamido]phenyl}acetyl-4-methoxypyrrolidin-2-yl)methoxycyclohexane-1-carboxylic acid ethyl ester (28)

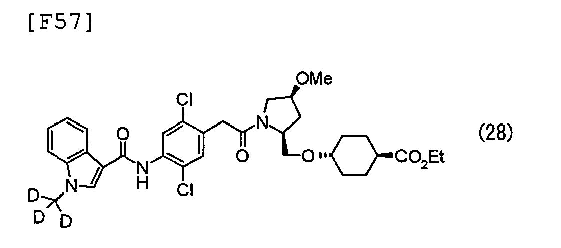

1,2-Dichloroethane (10 mL) was added to 1-methyl-d3-indole-3-carboxylic acid (604.0 mg, 3.389 mmol). Under stirring at -8°C, oxalyl chloride (438.4 µL, 5.112 mmol) was added to the mixture. The reaction mixture was stirred for 95 minutes at room temperature, and oxalyl chloride (290.7 µL, 3.389 mmol) was added to the reaction mixture, followed by stirring for 35 minutes at room temperature. The reaction mixture was concentrated to dryness under reduced pressure. The residue was dissolved in 1,2-dichloroethane (10 mL), and the solution was added to trans-4-[(2S,4S)-1-(4-amino-2,5-dichlorophenyl)acetyl-4-methoxypyrrolidin-2-yl]methoxycyclohexane-1-carboxylic acid ethyl ester (1.51 g, 3.098 mmol) in 1,2-dichloroethane (20 mL). The resultant mixture was refluxed for 11.5 hours under stirring. The reaction mixture was cooled to room temperature, and water (20 mL) was added thereto, followed by extraction with chloroform. The extract was dried over anhydrous sodium sulfate and then concentrated to dryness under reduced pressure. The residue was purified through silica gel column chromatography, and, from a fraction obtained through hexane:ethyl acetate (1:1 to 1:2, v/v), the title compound (28) was obtained as a pale yellow amorphous form (1.56 g, 2.409 mmol, 77.6%).
¹H-NMR(CDCl₃)δ: 1.15-1.34(5H,m), 1.35-1.55(2H,m), 1.93-2.15(6H,m), 2.17-2.33(1H,m), 3.25(1H,m), 3.31 and 3.34(total 3H,each s), 3.45-3.59(2H,m), 3.64-4.04(5H,m), 4.10(2H,q,J=7.2Hz), 4.19-4.33(1H,m), 7.30-7.46(4H,m), 7.80 and 7.81(total 1H,each s), 8.14(1H,m), 8.24(1H,m), 8.77 and 8.79(total 1H,each s).
MS (ESI)m/z 647(M⁺+1).

### Example 13

### trans-4-((2S,4S)-1-{2,5-Dichloro-4-[(indol-3-yl)carboxamido]phenyl}acetyl-4-methoxypyrrolidin-2-yl)methoxycyclohexane-1-carboxylic acid ethyl ester (29)

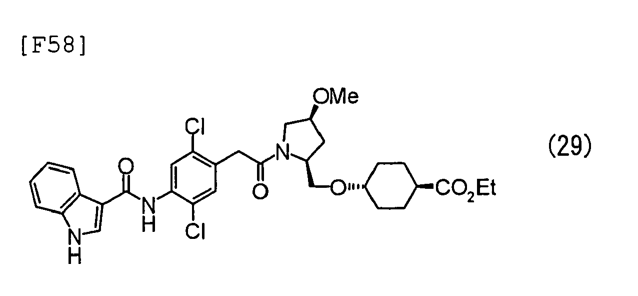

Oxalyl chloride (0.106 mL, 1.21 mmol) in 1,2-dichloroethane (3 mL) was added to indole-3-carboxylic acid (172 mg, 1.07 mmol) in 1,2-dichloroethane (3 mL) under stirring at 0°C. The reaction mixture was stirred for 1 hour at room temperature and concentrated to dryness under reduced pressure. The residue was dissolved in 1,2-dichloroethane (3 mL), and the solution was added to trans-4-[(2S,4S)-1-(4-amino-2,5-dichlorophenyl)acetyl-4-methoxypyrrolidin-2-yl]methoxycyclohexane-1-carboxylic acid ethyl ester (348 mg, 0.714 mmol) in 1,2-dichloroethane (15 mL) under stirring at 0°C. The reaction mixture was stirred for 10 hours under reflux conditions. The reaction mixture was cooled to room temperature, and water (20 mL) was added thereto, followed by extraction with 1,2-dichloroethane. The extract was washed with saturated brine, dried over anhydrous sodium sulfate, and concentrated to dryness under reduced pressure. The residue was purified through silica gel column chromatography, and, from a fraction obtained through use of chloroform:ethyl acetate (9:1 to 3:1, v/v), the title compound (29) was obtained as a crystalline powder (340 mg, 76%).
Melting point: 188-197°C.
¹H-NMR(CDCl₃)δ: 1.21-1.31(5H,m), 1.44-1.51(2H,m), 1.97-2.36(7H,m), 3.27(1H,m), 3.34 and 3.36(3H,each s), 3.50-4.35(11H,series of m), 7.26-7.30(3H,m), 7.41(1H,m), 7.69(1H,m), 8.06(1H,m), 8.17(1H,m), 8.62(1H,m), 9.54(1H,m).
Element analysis: Calcd for C₃₂H₃₇Cl₂N₃O₆: C, 60.95; H, 5.91; N,6.60.
Found: C,60.75; H,5.83; N,6.60.

### Example 14

(1) (4-Amino-2,5-dichlorophenyl)acetic acid ethyl ester hydrochloride (10 g, 35.1 mmol) was added to a flask, and methanol (30 mL, 3 v/w) and 2N aqueous sodium hydroxide (37.5 mL, 3.75 v/w) were added thereto, followed by stirring for 1 hour at about 70°C. Consumption of the starting materials were confirmed through HPLC, and the reaction mixture was left to cool.
   Concentrated hydrochloric acid (10 mL, 1 v/w) was added to the reaction mixture to thereby allow crystals to precipitate. After cooling with ice for 1 hour, the crystals that precipitated were collected through filtration with suction. The crystals were washed with water (30 mL) and dried under reduced pressure at 40°C, to thereby yield 4-amino-2,5-dichlorophenylacetic acid (7.3 g, yield: 95%). ¹H-NMR(400MHz, CD₃OD)δ: 7.17(s,1H), 6.84(s,1H), 4.88(brs,1H), 3.59(s,2H).

(2) trans-4-[(2S,4S)-1-(4-Amino-2,5-dichlorophenyl)acetyl-4-methoxypyrrolidin-2-yl]methoxycyclohexane-1-carboxylic acid ethyl ester

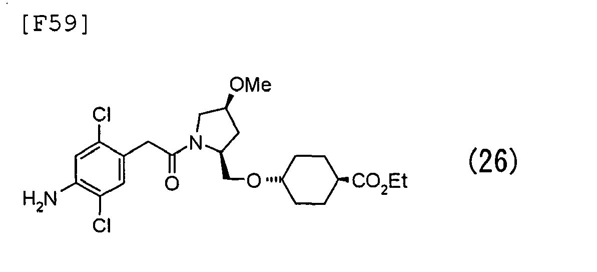

(2-1) (4-Amino-2,5-dichlorophenyl)acetic acid (6 g, 27.3 mmol), trans-4-[(2S,4S)-4-methoxypyrrolidin-2-yl)methoxycyclohexane-1-carboxylic acid ethyl ester (14.4 g, 1.02 mole-eq.), 1-hydroxybenzotriazole (4.43 g, 1.2 mole-eq.), and acetonitrile (120 mL, 20 v/w) were added to a flask. Under stirring, triethylamine (5.3 mL, 1.4 mole-eq.) was added to the mixture at room temperature. After the mixture had been confirmed to become a solution, 1-ethyl-3-(3-dimethylaminopropyl)carbodiimide hydrochloride (7.33 g, 1.4 mole-eq.) was added to the solution, followed by stirring. After completion of reaction had been confirmed through HPLC, water (80 mL, 15 v/w) was added to the reaction mixture, and the resultant mixture was stirred. Seed crystals were added to the reaction mixture to thereby allow crystals to precipitate, and the mixture was stirred for a while and cooled with ice.
The crystals that precipitated were collected through filtration with suction, sequentially washed with water:acetonitrile (1:1) (10 mL) and water (20 mL), and dried under reduced pressure at 40°C, to thereby yield first crop crystals of the compound represented by formula (26) (10.5 g, yield: 79%, quality: 99%).
The filtrate was concentrated under reduced pressure, and the residue was extracted with ethyl acetate. The extract was concentrated to dryness under reduced pressure. Acetonitrile (25 mL) was added to the residue, and the mixture was stirred. Water (20 mL) was added to the mixture, and seed crystals were added thereto, to thereby allow crystals to precipitate. The mixture was stirred for a while and cooled with ice. The precipitated crystals were collected through filtration with suction, sequentially washed with water:acetonitrile (1:1) (2 mL) and water (4 mL), and dried under reduced pressure at 40°C, to thereby second crop crystals of the compound represented by formula (26) (1.8 g, yield: 14%, quality: 96%).
¹H-NMR(400MHz, CDCl₃)δ: 1.24(dt,J=7.2,4Hz,3H), 1.45(t,J=12Hz,2H), 1.95-2.08(m,6H), 2.23(ddt,J=24.4,11.6,3.6Hz,2H), 3.31(d,J=11.2Hz,3H), 3.44-4.30(m,9H), 4.07(d,J=8.4Hz,2H), 4.10(ddd,J=14.4,7.2,2Hz,2H), 6.77(d,J=5.2Hz,1H), 7.17(d,J=4.8Hz,1H).

(2-2) Triethylamine (6.66 mL, 47.8 mmol) was added to a suspension of trans-4-[(2S,4S)-4-methoxypyrrolidin-2-yl]methoxycyclohexane-1-carboxylic acid ethyl ester (8.3 g, 29.0 mmol), (4-amino-2,5-dichlorophenyl)acetic acid (7.01 g, 31.9 mol), and butanol (4.52 g, 33.4 mmol) in dimethylacetic acid (75 mL). After the reaction mixture had turned to homogeneous, 1-ethyl-3-(3-dimethylaminopropyl)carbodiimide hydrochloride (6.37 g, 33.2 mmol) was added to the mixture. The resultant mixture was stirred for 4 hours at room temperature, and water (225 mL) was gradually added dropwise to the reaction mixture, followed by stirring for 3 hours at room temperature. The crystals that precipitated were collected through filtration, washed with 75% aqueous dimethylacetic acid (50 mL), and dried under reduced pressure, to thereby yield the compound represented by formula (26) (13.90 g, purity: 97.1%, purity-reduced isolation yield: 95.6%) as pale yellow crystals.
(3) trans-4-((2S,4S)-1-{2,5-Dichloro-4-[(1-methylindol-3-yl)carboxamido]phenyl}acetyl-4-methoxypyrrolidin-2-yl)methoxycyclohexane-1-carboxylic acid ethyl ester

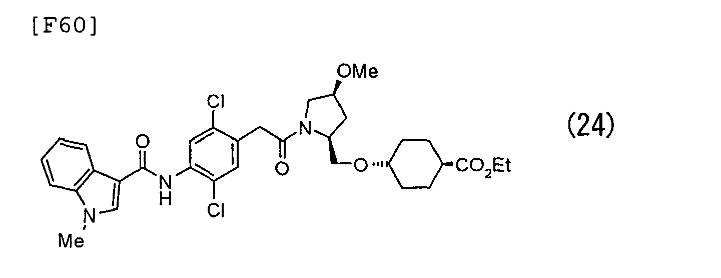

(3-1) 1-Methylindole-3-carboxylic acid (400 mg, 2.28 mmol) and toluene (4 mL, 10 v/w) were added to a flask, and the mixture was cooled with ice under nitrogen. Oxalyl chloride (243 µL, 1.2 mole-eq.) was added dropwise to the mixture, a catalytic amount of DMF was added to the resulting mixture, followed by stirring for 1 hour at room temperature. Triethylamine (413 µL, 1.3 mole-eq.) was added to the reaction mixture, and the compound represented by formula (26) (1.13 g, 1.02 mole-eq.) was added to the resulting mixture, followed by stirring for 4 hours at 75°C. After completion of reaction had been confirmed through HPLC, the reaction mixture was cooled, and isopropyl ether (7.2 mL, 18 v/w) was added to the reaction mixture. The resulting mixture was stirred for a while. The crystals that precipitated were collected through filtration, washed with isopropyl ether, and dried under reduced pressure at room temperature, to thereby yield the compound represented by formula (24) (1.3 g, 91%).

(3-2) DMF (70 mg) and thionyl chloride (3.07 mL, 42.7 mmol) were added to 1-methylindole-3-carboxylic acid (7.01 g, 40.0 mmol) in acetonitrile (70 mL), and the mixture was stirred for 30 minutes at room temperature. The solvent was removed under reduced pressure at an external temperature of 50°C. Acetonitrile (75 mL) and trans-4-[(2S,4S)-1-(4-amino-2,5-dichlorophenyl)acetyl-4-methoxypyrrolidin-2-yl]methoxycyclohexane-1-carboxylic acid ethyl ester (13.0 g, 26.7 mmol) were added to the residue, and the mixture was stirred for 2 hours at an internal temperature of 65°C. The reaction mixture was cooled to room temperature, and acetonitrile (50 mL) and water (130 mL) were gradually added dropwise thereto, followed by stirring overnight at room temperature. The crystals that precipitated were collected through filtration, washed with 50% aqueous acetonitrile (50 mL), and dried under reduced pressure, to thereby yield the compound represented by formula (24) (15.24 g, purity: 94.3%, purity-reduced isolation yield: 86.1%) as white crystals.

### Example 15

### Synthesis of (2,5-dichloro-4-nitrophenyl)acetic acid ethyl ester (30-a) and 2,5-dichloro-6-nitrophenylacetic acid ethyl ester (30-b)

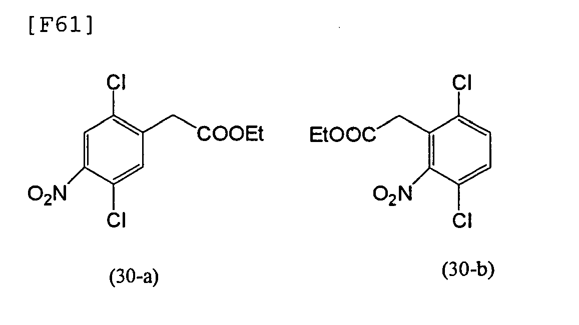

Sodium tert-butoxide (76.6 g, 0.797 mol) was dissolved in N,N-dimethylacetamide (250 mL) at room temperature, and the mixture was cooled to -10°C. A solution of 2,5-dichloronitrobenzene (50 g, 0.260 mol) and ethyl chloroacetate (42.5 mL, 0.399 mol) in N,N-dimethylacetamide (100 mL) was added dropwise to the solution. After completion of addition, the resultant mixture was stirred for 1 hour. After completion of reaction, the reaction mixture was diluted with ethyl acetate (500 mL), and 2N hydrochloric acid (400 mL) was added to the reaction mixture to thereby partition the mixture, followed by extraction with ethyl acetate (250 mL). The organic layers were combined together, sequentially washed with 2N hydrochloric acid (200 mL), water (200 mL), and saturated brine (200 mL), and concentrated under reduced pressure, to thereby yield a dark brown oil (87.0 g). The dark brown oil was found to be a mixture of the compound (30-a) and the compound (30-b) (the mixture containing the compound (30-b) in an amount of about 10%). 2-Propanol (105 mL) and water (35 mL) were added to the residue, and the mixture was stirred overnight. The crystals that precipitated were collected through filtration, to thereby yield the compound (30-a) (44.7 g, 0.161 mol, yield: 59.6%, HPLC purity: 100%) as yellow crystals. NMR data of the compound (30-a)
¹H-NMR(400MHz)δ: 1.29(t,3H,J=7.2Hz), 3.80(s,2H), 4.22(q,2H,J=7.2Hz), 7.53(s,1H), 7.98(s,1H)

### Example 16

### (2,5-Dichloro-nitrophenyl)acetic acid tert-butyl ester (31)

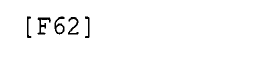

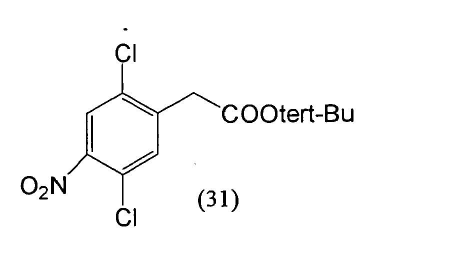

Sodium tert-butoxide (2.88 g) was dissolved in dimethylacetamide (18 mL) under cooling with ice, and a solution of 2,5-dichloronitrobenzene (1.92 g) and tert-butyl chloroacetate (2.26 g) in dimethylacetamide (18 mL) was added dropwise to the solution, followed by stirring for 2 hours at an internal temperature of 5°C or lower. 2N aqueous hydrochloric acid (23 mL) was added to the solution under cooling with ice, and the mixture was extracted three times with tert-butyl methyl ether (20 mL). The combined extract was washed with saturated brine, dried over magnesium sulfate, and subjected to filtration. The solvent was removed. The residue was subjected to silica gel column chromatography, to thereby give the compound represented by formula (31) (2.65 g, 86%) as an oil.
¹H NMR(CDCl₃,400MHz)δ: 1.49(s,9H), 3.72(s,2H), 7.51(s,1H), 7.97(s,1H)

### Example 17

### (2,5-Dichloro-nitrophenyl)acetic acid tert-butyl ester (31)

Lithium tert-butoxide (2.4 g) was dissolved in dimethylacetamide (18 mL) under cooling with ice. To the solution, a solution of 2,5-dichloronitrobenzene (1.92 g) and tert-butyl chloroacetate (2.26 g) in dimethylacetamide (18 mL) was added dropwise, and the mixture was stirred for 2 hours at an internal temperature of 5°C or lower. The reaction mixture was post-treated in a manner similar to that performed in Example 1, to thereby yield the title compound (2.71 g, 88%).

## Claims

1. A process for producing a compound represented by formula (III): 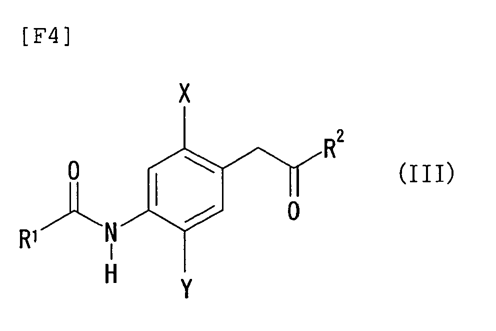 (wherein R¹ represents an aryl group which may be substituted, or a heteroaryl group which may be substituted; R² represents a linear or branched lower alkoxy group which may be substituted, an aralkyloxy group which may be substituted, a phenoxy group, or a group represented by formula (a): 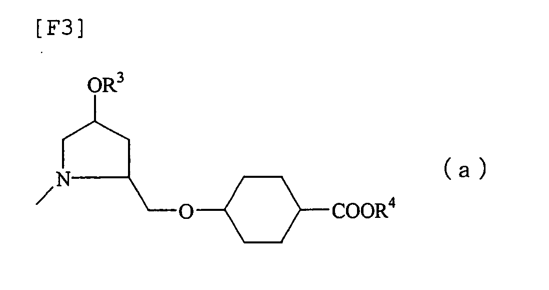 (wherein R³ represents a linear or branched lower alkyl group which may be substituted; and R⁴ represents a linear or branched lower alkyl group which may be substituted, or an aralkyl group which may be substituted); X represents a hydrogen atom or a halogen atom; and Y represents a halogen atom or a lower alkoxy group), **characterized by** comprising reacting a compound represented by formula (I): 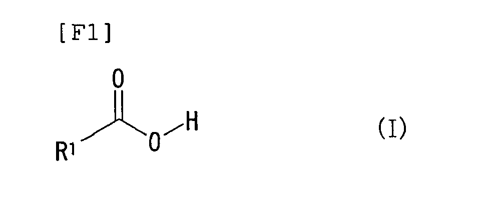 (wherein R¹ has the same meaning as defined above) with a chlorinating agent and a compound represented by formula (II): 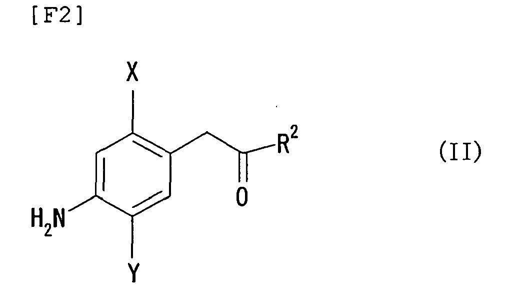 (wherein R² has the same meaning as defined above) or a salt thereof under acidic conditions without addition of a base.

2. The process according to claim 1, wherein R¹ represents a 1-methylindolyl group.

3. The process according to claim 1 or 2, wherein the chlorinating agent is oxalyl chloride or thionyl chloride.

4. The process according to any one of claims 1 to 3, wherein R² represents a linear or branched lower alkoxy group.

5. The process according to any one of claims 1 to 3, wherein R² represents a group represented by formula (a) wherein R³ represents a methyl group, and R⁴ represents a linear or branched lower alkyl group.

6. The process according to any one of claims 1 to 5, wherein X represents a chlorine atom or fluorine atom.

7. The process according to any one of claims 1 to 6, wherein X represents a chlorine atom, Y represents a chlorine atom, and R¹ represents a 1-methylindolyl group.

8. A hydrochloric acid salt of a compound represented by formula (IV).

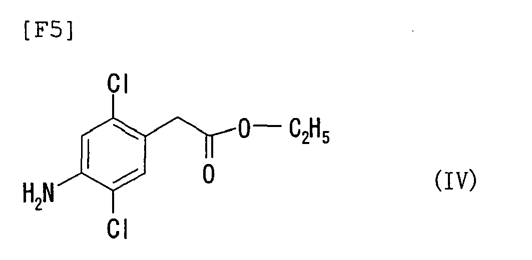

9. The process according to any one of claims 1 to 7, wherein the compound represented by formula (II) or a salt thereof is a hydrochloric acid salt as recited in claim 8.

10. A process for producing a compound represented by formula (VII): 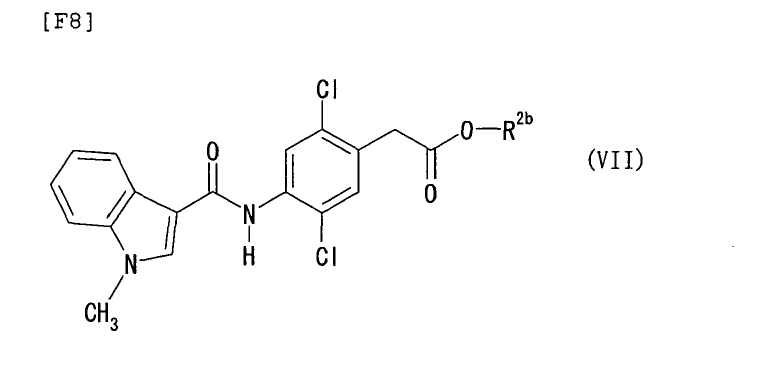 (wherein R^{2b} represents a linear or branched lower alkyl group which may be substituted, an aralkyl group which may be substituted, or a phenyl group), **characterized by** comprising reacting a compound represented by formula (V): 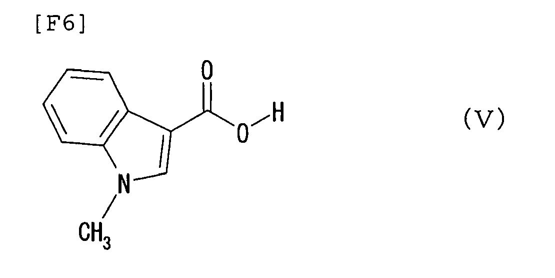 with a chlorinating agent and a compound represented by formula (VI): 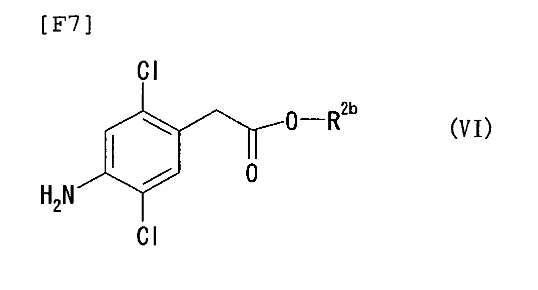 (wherein R^{2b} has the same meaning as defined above) or a salt thereof under acidic conditions without addition of a base.

11. A process for producing a compound represented by formula (IX): 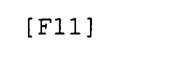 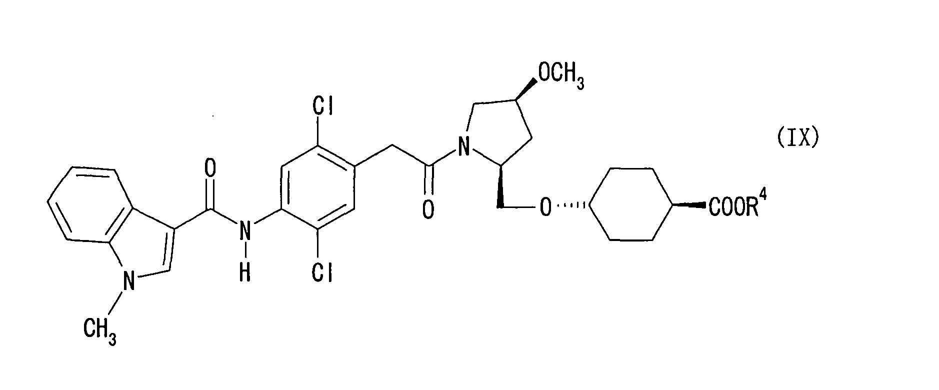 (wherein R⁴ represents a linear or branched lower alkyl group which may be substituted, or an aralkyl group which may be substituted), **characterized by** comprising reacting a compound represented by formula (V): 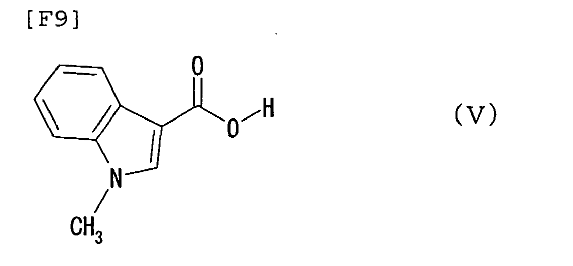 with a chlorinating agent and a compound represented by formula (VIII): 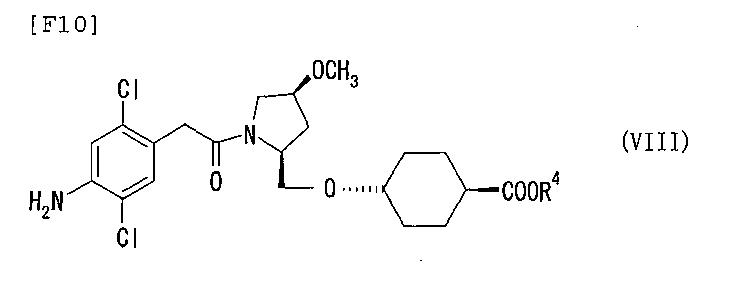 (wherein R⁴ has the same meaning as defined above) or a salt thereof under acidic conditions without addition of a base.

12. A process for producing a compound represented by formula (XII): 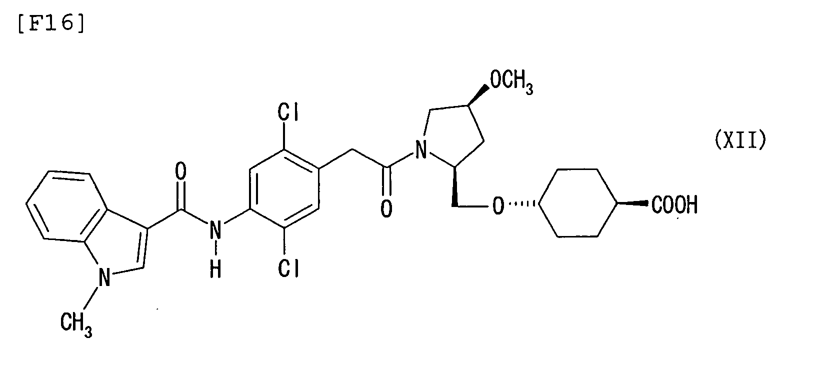 or a salt thereof, or a hydrate of the compound or the salt, **characterized by** comprising reacting a compound represented by formula (V): 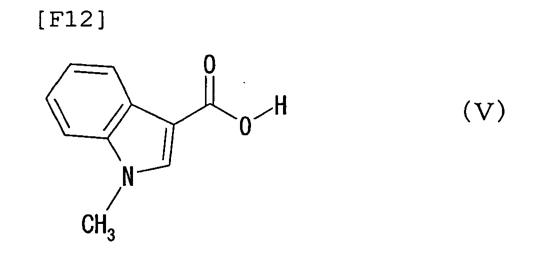 with a chlorinating agent and a compound represented by formula (VI): 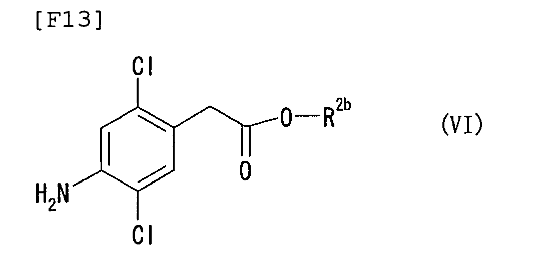 (wherein R^{2b} represents a linear or branched lower alkyl group which may be substituted, or an aralkyl group which may be substituted, or a phenyl group) or a salt thereof under acidic conditions without addition of a base; optionally hydrolyzing the product to thereby yield a compound represented by formula (X): 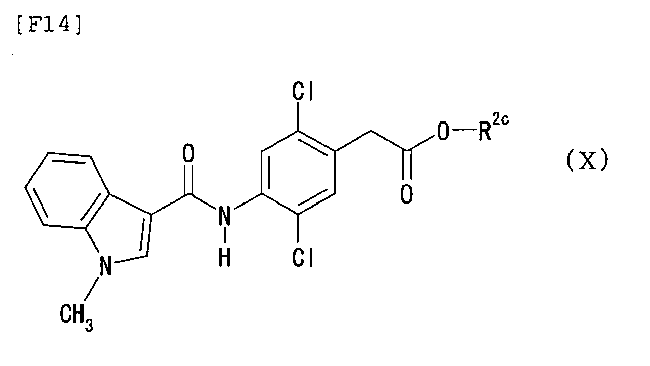 (wherein R^{2c} represents a hydrogen atom, a linear or branched lower alkyl group which may be substituted, an aralkyl group which may be substituted, or a phenyl group); reacting the compound represented by formula (X) with a compound represented by formula (XI): 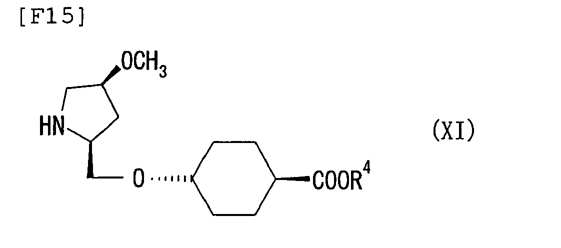 (wherein R⁴ represents a linear or branched lower alkyl group which may be substituted, or an aralkyl group which may be substituted); and hydrolyzing the product.

13. A process for producing a compound represented by formula (XII): 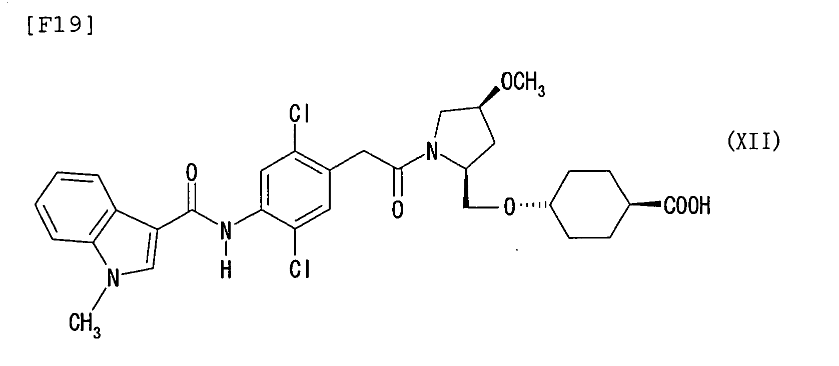 or a salt thereof, or a hydrate of the compound or the salt, **characterized by** comprising reacting a compound represented by formula (V): 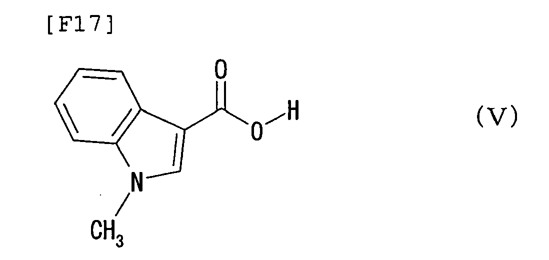 with a chlorinating agent and a compound represented by formula (VIII): 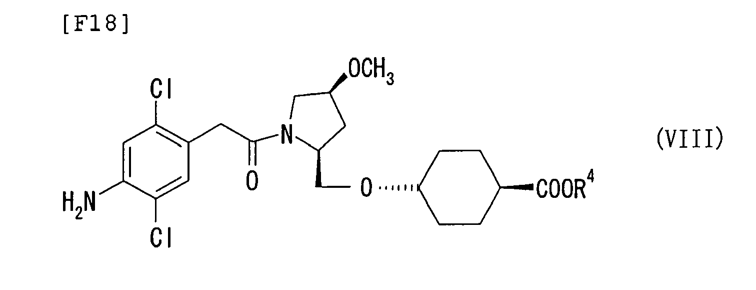 (wherein R⁴ represents a linear or branched lower alkyl group which may be substituted, or an aralkyl group which may be substituted) or a salt thereof under acidic conditions without addition of a base; and hydrolyzing the product.

14. A process for producing a compound represented by formula (XIII) : 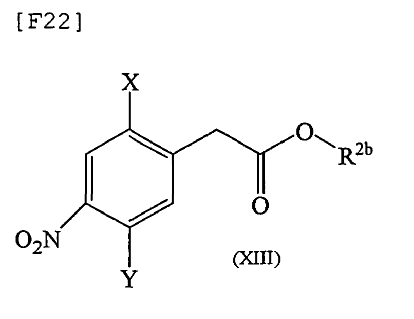 (wherein X represents a hydrogen atom or a halogen atom; Y represents a halogen atom or a lower alkoxy group; and R^{2b} represents a linear or branched lower alkyl group which may be substituted, or an aralkyl group which may be substituted, or a phenyl group), **characterized by** comprising reacting a compound represented by formula (XIV): 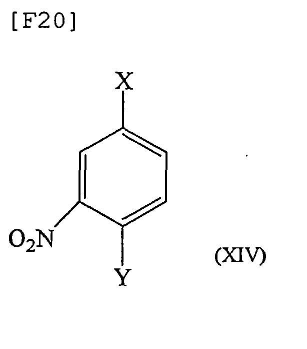 (wherein X and Y have the same meanings as defined above) with a compound represented by formula (XV): 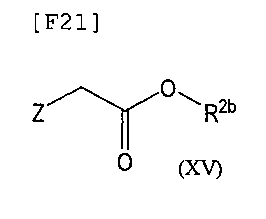 (wherein Z represents a halogen atom, a phenylthio group, an alkoxy group, or an amino group; and R^{2b} has the same meaning as defined above) in a solvent in the presence of a base.

15. The process according to claim 14, wherein each of X and Y in formulas (XIII) and (XIV) represents a chlorine atom.

16. The process according to claim 15, wherein R^{2b} in formulas (XIII) and (XV) represents a tert-butyl group.

17. A compound represented by formula (XIII): 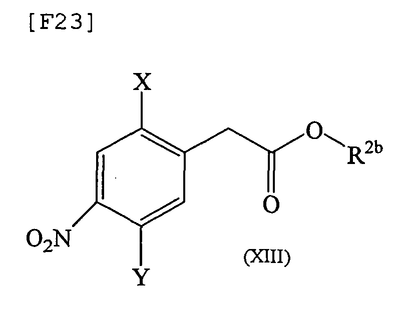 (wherein X represents a hydrogen atom or a halogen atom; Y represents a halogen atom or a lower alkoxy group; and R^{2b} represents a linear or branched lower alkyl group which may be substituted, an aralkyl group which may be substituted, or a phenyl group), a salt thereof, or a solvate of the compound or the salt.

18. The compound according to claim 17, a salt thereof, or a solvate of the compound or the salt, wherein each of X and Y in formula (XIII) represents a chlorine atom.

19. The compound according to claim 18, a salt thereof, or a solvate of the compound or the salt, wherein R^{2b} in formula (XIII) represents a tert-butyl group.

20. The process according to any one of claims 1 to 7, wherein the compound represented by formula (II) is a compound produced through reduction of the nitro group of the compound represented by formula (XIII) produced through the process according to claim 14, a salt thereof, or the solvate of the compound or the salt.

21. The process according to claim 10 or 12, wherein the compound represented by formula (VI) is a compound produced through reduction of the nitro group of the compound represented by formula (XIII) produced through the process according to claim 15 or 16, a salt thereof, or the solvate of the compound or the salt.
